(19) Europäisches Patentamt
European Patent Office
Office européen des brevets

(11) Publication number: **0 404 935 B1**

(12) **EUROPEAN PATENT SPECIFICATION**

(45) Date of publication of patent specification: **04.01.95**

(51) Int. Cl.⁶: **C07K 7/08**, C07K 7/10, C12Q 1/70, G01N 33/569

(21) Application number: **90903508.1**

(22) Date of filing: **16.01.90**

(86) International application number: **PCT/US90/00260**

(87) International publication number: **WO 90/08162 (26.07.90 90/17)**

(54) **SYNTHETIC PEPTIDE COMPOSITIONS WITH IMMUNOREACTIVITIES TO ANTIBODIES TO HTLV-1.**

(30) Priority: **13.01.89 US 297635**

(43) Date of publication of application: **02.01.91 Bulletin 91/01**

(45) Publication of the grant of the patent: **04.01.95 Bulletin 95/01**

(84) Designated Contracting States: **AT BE CH DE DK ES FR GB IT LI LU NL SE**

(56) References cited:
**EP-A- 0 345 792**
**WO-A-86/01834**
**WO-A-89/06543**
**US-A- 4 833 071**

(73) Proprietor: **United Biomedical Inc.**
**2 Nevada Drive**
**Lake Success**
**New York 11042 (US)**

(72) Inventor: **YANG, Chang, Yi**
**2 Nevada Drive**
**Lake Success, NY 11042 (US)**

(74) Representative: **Hansmann, Axel et al**
**Patent- und Rschtsanwälte**
**HANSMANN VOGESER DR. BOECKER ALBER DR. STRYCH**
**Albert-Rosshaupter-Strasse 65**
**D-81369 München (DE)**

EP 0 404 935 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

### INTRODUCTION

Priority is claimed based on United States Application Serial Number 07/297,635, filed January 13, 1989.

Human T-cell leukemia virus subgroup I (now designated HTLV-1) is a retrovirus causatively linked to certain adult lymphoid malignancies, notably adult T-cell leukemia-lymphoma (ATL) (1-3). Antibodies that react with HTLV-1 proteins have been found in sera of ATL patients. These HTLV-1 antibodies recognize both the gag core antigens and the envelope proteins of the HTLV-1 virus (4,5). Human T cell lymphotropic virus type III (once known as HTLV-III, now designated Human Immunodeficiency Virus or HIV) is a retrovirus causatively linked to Acquired Immune Deficiency Syndrome (AIDS) and AIDS related complex (ARC). Antibodies that react with HIV proteins have been found in the sera of AIDS and ARC patients. These HIV antibodies recognize both the gag core antigens and envelope proteins of the HIV virus. In the United States, the disease AIDS is far more prevalent than ATL, with some individuals seropositive for HIV also being seropositive for HTLV-1.

The present invention relates to highly sensitive methods for the detection of antibodies to HTLV-1 in body fluids by the use of synthetic peptide compositions. The present invention further relates to a highly sensitive method for the simultaneous detection of antibodies to HTLV-1 and HIV in body fluids by the use of synthetic peptide compositions. One peptide composition comprises peptides having amino acid sequences corresponding to segments of the external (extracellular) portion of the HTLV-1 envelope protein, designated gp 46, and may further comprise peptides having amino acid sequences corresponding to segments of the transmembrane portion of the HTLV-1 envelope protein, designated gp21. These sequences have been found to be highly immunoreactive to antibodies in the sera of patients with ATL. Such peptide compositions are also useful for the production of a vaccine for ATL by stimulating the production of antibodies to HTLV-1, which provide protection against HTLV-1 infection in healthy mammals, including humans. Furthermore, a peptide composition comprising peptides with amino acid sequences corresponding to portions of HTLV-1 envelope proteins may be used in conjuction with a peptide composition comprising peptides with amino acid sequences corresponding to portions of the HIV envelope and core proteins for the simultaneous detection of antibodies to HTLV-1 and HIV.

More specifically, the present invention is directed to peptide compositions, useful for the detection of HTLV-1 antibodies and the diagnosis of ATL, which comprise peptides selected from the group consisting of chemically synthesized peptides containing about thirty-four, forty, thirty-eight, twenty, twenty-four and sixteen amino acids, or their analogues, in a prescribed sequence; analogues, segments, mixtures, conjugates and polymers thereof. The invention is further directed to the use of an HTLV-1 peptide composition in conjunction with an HIV peptide composition which comprises peptides selected from the group consisting of chemically synthesized peptides containing about twenty-one, nineteen, eleven and sixteen amino acids, sequence; analogues, segments, mixtures, conjugates and polymes thereof, for the simultaneous detection of antibodies to HTLV-1 and HIV in human body fluids.

The detection methods include an enzyme-linked immunoadsorbent assay (ELISA), multi-dot, multi-line, or multi-square blotting on nitrocellulose paper, and a passive hemagglutination assay using the peptides as the solid phase antigens. The preferred detection method is by ELISA.

### BACKGROUND OF THE INVENTION

The human T cell leukemia-lymphoma viruses (HTLV) are a family of related retroviruses originally isolated from patients with T cell lymphoma and cutaneous manifestations. A particular subgroup of the family, type I, now known as HTLV-1, has been causatively linked to malignancies which share clinical and epidemiologic features with the disease called adult T-cell leukemia-lymphoma (ATL) which occurs in certain regions of Japan (6-9), the Caribbean Basin (10,11) and the southwestern United States (12).

Although the mechanism of transmission of HTLV-1 is currently unknown, horizontal transmission of HTLV is clearly implicated by molecular and epidemiologic analyses (13,14). HTLV-1 seropositivity in regions endemic for ATL is elevated overall in the general population and further elevated among close family members of patients and in the recipients of blood transfusions (15,16).

This means that there is an urgent need for a safe, reliable and sensitive test to screen each blood sample before its inclusion in blood banks and to isolate blood donations derived from HTLV-1 infected individuals to avoid the inadvertent spread of the virus among patients who must receive blood transfusions, e.g. hemophiliacs and surgical patients.

The complete nucleotide sequence of the HTLV-1 virus was reported in 1983 (17). This report elucidated the structure of the HTLV-1 virus at both the DNA level and the predicted protein level and permitted further serological studies of different epitopes which may be present on the HTLV-1 virus.

Simultaneously, Dr. Carl Saxinger at National Cancer Institute reported the use of the isolated HTLV-1 virus as a solid-phase immunoadsorbent for the development of an enzyme immunoassay for the detection of HTLV-1 antibodies in the African population (18).

It was further reported by Samuel et al. (19) that a combined cloning and expression system in E. Coli had been used to identify HTLV-1 DNA encoded glycoproteins which reacted immunologically with antibodies in sera from ATL patients. HTLV-1 DNA encoding the envelope protein was cleaved into fragments and inserted into an expression vector. The expression vectors were introduced into an E. Coli host by transformation. One clone, designated as pKS400, produced an envelope protein product found to be suitable for use as an immunoadsorbent to screen a group of 28 coded sera. Antibodies that recognized the bacterially synthesized HTLV-1 envelope protein sequences were found in all sera that had been shown to have antibodies to HTLV-1 by an ELISA assay with disrupted virions as the antigen (18).

Slamon et al, Application No. PCT/US 85/01803, published on March 27, 1986 under Publication No. W086/01834, described polypeptides associated with immunogenic sites of HTLV-1 as expression products of the X region of HTLV-1, a highly conserved region located between env and the 3' LTR of the virus. The proteins, with a molecular weight of between 37 kd and 40 kd, were cloned and expressed as fusion proteins in E. coli. The resulting products were purified and used in liquid phase immunoprecipitation tests to screen sera. The results indicated an accuracy of from about 77% to 87%. (20)

Synthetic peptides increasingly have been used to map antigenic or immunogenic sites on the surface of proteins and as possible vaccines. The named inventor and a colleague previously have taken this approach to identify and characterize highly antigenic epitopes on the envelope proteins of HTLV-III and to develop sensitive and specific immunoassays for the detection of antibodies to HTLV-III (now designated HIV) (21) See also U.S. Patent Serial Number 4,735,896, issued April 5, 1988, the contents of which are, hereby, fully incorporated by reference. (22). A similar approach is employed in this invention to select and identify highly antigenic epitopes in HTLV-1. In selecting regions of the envelope protein for epitope analysis, several strategies were employed. First, regions that exhibited a relatively high conservation of amino acid sequence between HTLV-1 and HTLV-2 were sought. Second, multiple overlapping linear peptides covering whole regions of gp21, the transmembrane portion of the HTLV-1 envelope protein (See Figure 1), were synthesized and characterized. Third, multiple overlapping linear peptides covering whole regions of gp 46, the external portion of the HTLV-1 envelope protein (See Figure 1), were synthesized and characterized. Three peptides, from the transmembrane portion, with the following sequences (See Figure 2), and a mixture thereof, were found to be highly immunoreactive with sera from patients with ATL:

GLDLLFWEQGGLCKALQEQC-NH2 (I)

QNRRGLDLLFWEQGGLCKALQEQC-NH2 (II)

NRRGLDLLFWEQGGLC-NH2 (III)

and three peptides, from the external portion, with the following sequences, and a mixture thereof, were also found to be highly immunoreactive with sera from patients with ATL (See Figure 3):

APPLLPHSNLDHILEPSIPWKSKLLTLVQLTLQS-$NH_2$ (IV)

SSTPLLYPSLALPAPHLTLPFNWTHCFDPQIQAICSSPCH-$NH_2$ (V)

CFDPQIQAIVSSPCHNSLILPPFSLSPVPTLGSRSRRA-$NH_2$ (VI)

wherein:

| | |
|---|---|
| A = Ala = alanine, | G = Gly = glycine, |
| R = Arg = arginine, | I = Ile = isoleucine, |
| D = Asp = aspartic acid, | F = Phe = phenylalanine, |
| N = Asn = asparagine, | S = Ser = serine, |
| Q = Gln = glutamine, | W = Trp = tryptophan, |
| E = Glu = glutamic acid, | Y = Tyr = tyrosine, |
| L = Leu = leucine, | V = Val = valine, |
| K = Lys = lysine, | C = Cys = cysteine |
| H = His = histidine | P = Pro = proline |
| T = Thr = threonine | |

Assays for antibodies to HTLV-1 based upon chemically synthesized peptides show several advantages over assays utilizing whole disrupted virus or bacterially produced immunoadsorbents. The peptides can

easily be synthesized in gram quantities by using automated solid-phase methods, thus providing a reproducible antigen of high integrity with consistent yields. Isolation of antigens from biological systems precludes such reproducibility. More importantly, non-specific reactivities seen in non-HTLV-1 infected individuals are likely due to the heterogeneity of the preparations used for assay. This is particularly true for assays using either the whole virus or Escherichia coli-derived recombinant products as immunoadsorbents. In these processes, the major histocompatibility antigens or endogenous bacterial proteins of the host cells are frequently copurified with the desired antigen virus or protein. Since antibodies to these contaminating antigens are frequently found in normal individuals, false-positive results cannot be eliminated by using current antigen isolation processes.

The assay of the present invention thus clearly eliminates the false-positive reactions encountered in the other methods and, at the same time, shows a high sensitivity to truly positive sera by the substantially increased signal-to-noise ratio. This increased signal-to-noise ratio likely results from the purity of the immunodsorbent.

Furthermore, up to the present, no viable vaccine or method to provide protection against HTLV-1 has been reported. Utilization of deactivated virus provokes fears of contracting the disease, preventing its acceptability and use.

Similarly, the development of monoclonal and polyclonal antibodies to HTLV-1 in mammals involves the use of HTLV-1 as the immunogen, presenting unacceptable risks in the procedure.

It is, therefore, an objective of the present invention to develop a detection or diagnostic procedure that does not require the use of the virus or lysates thereof as a test reagent.

A further objective is to develop a test procedure that is highly sensitive and accurate.

Another objective is to develop a test that is highly sensitive so that very little test reagent or body fluid is needed to obtain an accurate result.

A further objective is to prepare a test reagent by chemical means. The synthetic reagent can then be used to detect the presence of antibodies to HTLV-1 in body fluids and diagnose ATL, thereby avoiding the danger of exposure to the virus or segments thereof and the unnecessary proliferation of the virus.

Another objective is to develop a vaccine which, when introduced into healthy mammals, including humans, will stimulate production of antibodies to HTLV-1, thereby providing protection against HTLV-1 infection.

A further objective is to provide a non-viral immunogen which can be used in mammals for the development of monoclonal and polyclonal antibodies to HTLV-1.

Another objective is to develop a diagnostic procedure for the simultaneous detection of antibodies to HTLV-1 and antibodies to HIV.

REFERENCES


1. B.J. Poiesz., et al., Proc. Natl Acad. Sci. USA., 77:7415 (1980).
2. B. J. Poiesz., F.W. Ruscetti,M., S. Reitz., V.S.Kalyanaraman, R. Gallo, Nature (London) 294:268 (1981).
3. R.C. Gallo et al., Proc. Natl. Acad. Sci. USA., 79:5680 (1982).
4. M.Essex et al., Science, 221:1061 (1983).
5. P. Clapham, K. Napy, R. A. Weiss, Proc. Natl. Acad. Sci. 81:2886 (1984).
6. R. C. Gallo et al., Cancer Res., 43: 3892 (1983).
7. R. C. Gallo, Cancer Surveys, L. M. Franks et al. Eds, (University Press, Oxford, in press).
8. W. A. Blattner, K. Tokatsuki, R. C. Gallo. J.Am. Med. Assoc., 250:1074 (1983).
9. K. Takatsuki, J. Uchiyama, K. Sagawa, J. Yodoi, Topics in Hematology, S. Seno, F. Takaku, S. Irino, Eds.(Excerpta Medica, Amersterdam, 1977) p73.
10. W. A. Blattner et al., Int. J. Cancer, 30:257 (1982)
11. D. Catovsky et al., Lancet, 1982-I, 639 (1982).
12. D. W. Blayney et al., J. Am. Med. Assoc., 250:1048 (1983).
13. M. Robert-Guroff, F. W. Ruscetti, L. W. Posner, B. J.Poiesz, R. C. Gallo, J. Exp. Med., 154:1957 (1981).
14. R. C. Gallo et al., Proc. Natl. Acad. Sci. USA., 79:5680 (1981).
15. M. Robert-Guroff et al., J. Exp. Med., 157:248 (1983).
16. M. Shimoyama et al, Jpn. J. Clin. Oncol., 12:109 (1982).
17. M. Seiki, S. Hattori, Y. Hirayama, M. Yoshida Proc. Natl Acad. Sci. USA., 80:3618 (1983).
18. Saxinger, C. W. et al., Science, 225:1473 (1984).
19. Samuel, K.P. et al., Science, Nov. 30, 1984.
20. Slamon et al., PCT Patent Publication No. W086/01834.

21. Wang, J.J-G, Steel, S., Wisniewolski, R. and Wang, C.Y. Proc. Natl. Acad. Sci. USA, 83, pp 6159-6163 (August 1986).
22. U.S. Patent No. 4,735,896. issued April 5, 1988 to Chang Y. Wang and James G. Wang.
23 Liu, Fu-Tong et al., Biochemistry, 18, pp. 690-697 (1979).

## BRIEF DESCRIPTION OF THE INVENTION

According to the present invention, six peptides, each arranged in a specific sequence, have been made by solid phase peptide synthesis. These peptides have been found to be useful in a highly sensitive and accurate method for the detection of antibodies to HTLV-1 in sera and body fluids and in the diagnosis of ATL. These peptides have also been found to be useful in stimulating production of antibodies to HTLV-1 in healthy mammals such as Balb/c mice.

According to the present invention, a peptide composition useful for the detection of antibodies to HTLV-1 and diagnosis of ATL comprises a peptide selected from the group of peptides comprising:

GLDLLFWEQGGLCKALQEQC-Z (I)
QNRRGLDLLFWEQGGLCKALQEQC-Z (II)
NRRGLDLLFWEQGGLC-Z (III)
APPLLPHSNLDHILEPSIPWKSKLLTLVQLTLQS-Z (IV)
SSTPLLYPSLALPAPHLTLPFNWTHCFDPQIQAICSSPCH-Z (V)
CFDPQIQAIVSSPCHNSLILPPFSLSPVPTLGSRSRRA-Z (VI)

wherein Z is -OH or $-NH_2$, analogues, segments, mixtures conjugates and polymers thereof, wherein:

| | |
|---|---|
| A = Ala = alanine, | G = Gly = glycine, |
| R = Arg = arginine, | I = Ile = isoleucine, |
| D = Asp = aspartic acid, | F = Phe = phenylalanine, |
| N = Asn = asparagine, | S = Ser = serine, |
| Q = Gln = glutamine, | W = Trp = tryptophan, |
| E = Glu = glutamic acid, | Y = Tyr = tyrosine, |
| L = Leu = leucine, | V = Val = valine, |
| K = Lys = lysine, | C = Cys = cysteine. |
| H = His = histidine | P = Pro = proline |
| T = Thr = threonine | |

The highly sensitive and accurate method of detecting antibodies to HTLV-1 in body fluids and diagnosis of ATL comprises the following steps:

A. Preparing a peptide composition comprising a peptide selected from the group having the following amino acid sequences:

GLDLLFWEQGGLCKALQEQC-Z (I)
QNRRGLDLLFWEQGGLCKALQEQC-Z (II)
NRRGLDLLFWEQGGLC-Z (III)
APPLLPHSNLDHILEPSIPWKSKLLTLVQLTLQS-Z (IV)
SSTPLLYPSLALPAPHLTLPFNWTHCFDPQIQAICSSPCH-Z (V)
CFDPQIQAIVSSPCHNSLILPPFSLSPVPTLGSRSRRA-Z (VI)

wherein Z is -OH or $-NH_2$, analogues, segments, mixtures, conjugates and polymers thereof; and

B. Using about 0.01 $\mu$g to about 20 $\mu$g per test in a buffer at a pH of about 7 to 10, of the peptide composition as the antigen in an immunoassay procedure.

Further, according to the present invention, the peptides by themselves, or when coupled to a protein or a polymer carrier, or when polymerized to homo or hetero dimers or higher oligomers by cysteine oxidation, induced disulfide cross linking, or when polymerized to homo or hetero dimers or higher oligomers by use of homo or hetero functional multivalent cross linking reagents, or when directly synthesized onto a polyvalent lysine resin, can be used to stimulate production of antibodies to HTLV-1 in healthy mammals, including humans. The method comprises introducing an effective amount of the peptide composition including a mixture of these six peptides, conjugated to a carrier, such as human serum albumin, or as a polymer, into the body of a healthy mammal by intraperitoneal or subcutaneous injection.

In addition, according to the present invention, a peptide composition useful for the detection of antibodies to HTLV-1 may be used in conjunction with peptide compositions useful for the detection of antibodies to HIV-1 and HIV-2, for the simultaneous detection of infection by both HTLV-1 and HIV-1 and

HIV-2. Peptide compositions useful for the detection of antibodies to HIV-1 and HIV-2 comprise chemically synthesized peptides of the following amino acids, or their analogues, in the prescribed sequences wherein the sequence for HIV-2 is an analogue of peptide VII and peptide VIII:

HIV-1

RILAVERYLKDQQLLGIWGCS-Z (VII)
IWGCSGKLICTTAVPWNAS-Z (VIII)
IVRMYSPTSIL-Z (IX)

HIV-2

DQARLNSWGCAFRQVC (X)
wherein Z is -OH or $-NH_2$, and include analogues, segments, mixtures and polymers thereof, wherein:

| | |
|---|---|
| A = Ala = alanine, | G = Gly = glycine, |
| R = Arg = arginine, | I = Ile = isoleucine, |
| D = Asp = aspartic acid, | F = Phe = phenylalanine, |
| N = Asn = asparagine, | S = Ser = serine, |
| Q = Gln = glutamine, | W = Trp = tryptophan, |
| E = Glu = glutamic acid, | Y = Tyr = tyrosine, |
| L = Leu = leucine, | V = Val = valine, |
| K = Lys = lysine, | C = Cys = cysteine. |
| H = His = histidine | P = Pro = proline |
| T = Thr = threonine | |
| M = Met = methionine | |

The underlined amino acids indicate the residues shared between various isolates. For HIV-2 peptide X, substitutions were made in the envelope protein amino acid sequence that would be predicted from the nucleotide sequence.

## BRIEF DESCRIPTION OF THE DRAWINGS

Figure 1 shows and compares the amino acid sequences of the HTLV-1 and HTLV-2 envelope proteins.

Figure 2 shows the amino acid sequences of the chemically synthesized peptides described herein.

Figure 3 is a histogram depicting the immunoreactivties described herein, with sera from ATL patients.

Figure 4 is a histogram depicting the immunoreactivties of the peptides described herein with sera from patients with HIV infection, patients with ATL, and random blood donors.

Figure 5 is a histogram depicting the simultaneous detection of antibodies to HTLV-1 and HIV (1 and 2) by an enzyme immunoassay employing a mixture of seven chemically synthesized peptides described herein.

## DETAILED DESCRIPTION OF THE INVENTION

In accordance with the present invention, six peptides have been chemically synthesized for the detection of antibodies to HTLV-1 in body fluids and the diagnosis of ATL, for the vaccination of healthy mammals by stimulating the production of antibodies to HTLV-I in healthy mammals, and for the development of both monoclonal and polyclonal antibodies to HTLV-1 in mammals. These six peptides are arranged in the following sequences:

GLDLLFWEQGGLCKALQEQC-Z (I)
QNRRGLDLLFWEQGGLCKALQEQC-Z (II)
NRRGLDLLFWEQGGLC-Z (III)
APPLLPHSNLDHILEPSIPWKSKLLTLVQLTLQS-Z (IV)
SSTPLLYPSLALPAPHLTLPFNWTHCFDPQIQAICSSPCH-Z (V)
CFDPQIQAIVSSPCHNSLILPPFSLSPVPTLGSRSRRA-Z (VI)
wherein Z is -OH or $-NH_2$.

These peptides may comprise analogues or segments, i.e. longer or shorter peptide chains by having more amino acids added to the terminal amino acids, e.g., -Gly-,-Gln-,-Asn- and -Cys- of the above sequence, or by amino acids removed from either terminal end. These peptides may also comprise conjugates, i.e., they may be coupled to carrier proteins such as bovine serum albumin (BSA) or human serum albumin (HSA). Furthermore, these peptides may comprise polymers, i.e, they may be synthesized on a polymeric resin, such as a branching octameric lysine resin. It is expected that as long as the peptide immunoreactivities recognizable by the dominant antibodies to HTLV-1 are preserved, analogues of the synthetic peptide may also comprise substitutions, insertions and/or deletions of the recited amino acids of the above sequence.

In addition, to accomodate strain-to-strain variations among different isolates, adjustments for conservative substitutions and selection among the alternatives where non-conservative substitutions are involved, may be made in the prescribed sequences.

The amino acid sequences of the polypeptides useful as test reagents for the detection of antibodies to HTLV-1 in body fluids and diagnosis of ATL are selected to correspond to a partial segment of the amino acid sequence of the HTLV-1 virus designated as gp21, and to a partial segment of the amino acid sequence of the HTLV-1 virus designated as gp46, both parts of gp 61, which defines the envelope protein of the HTLV-1 virus.

The peptides useful as solid phase immunoadsorbents for the detection of antibodies HTLV-1 were synthesized by the "classical" Merrifield method of solid phase peptide synthesis using t-Boc-amino acids to correspond to the following amino acid sequences:

GLDLLFWEQGGLCKALQEQC-Z (I)
QNRRGLDLLFWEQGGLCKALQEQC-Z (II)
NRRGLDLLFWEQGGLC-Z (III)
APPLLPHSNLDHILEPSIPWKSKLLTLVQLTLQS-Z (IV)
SSTPLLYPSLALPAPHLTLPFNWTHCFDPQIQAICSSPCH-Z (V)
CFDPQIQAIVSSPCHNSLILPPFSLSPVPTLGSRSRRA-Z (VI)

wherein Z is -OH or $-NH_2$.

Analogues of these six peptides can be prepared by varying the amino acid sequences either by adding, subtracting substituting, or deleting desired t-Boc-amino acid(s).

Following completion of assembly of the desired blocked peptide on the resin, the peptide-resin is treated with anhydrous hydrofluoric acid to cleave the benzyl ester linking the peptide to the resin in order to liberate the peptide. Functional groups of amino acids which are blocked during synthesis by benzyl-derived blocking groups are also cleaved from the peptide simultaneously. The free peptide is then analyzed and purified by high performance liquid chromatography (HPLC) and characterized biochemically by amino acid analysis.

Similarly, synthesis of these peptides that have an amide group on its C-terminal end can be achieved by using 4-methylbenzhydrylamine resin according to the following scheme:

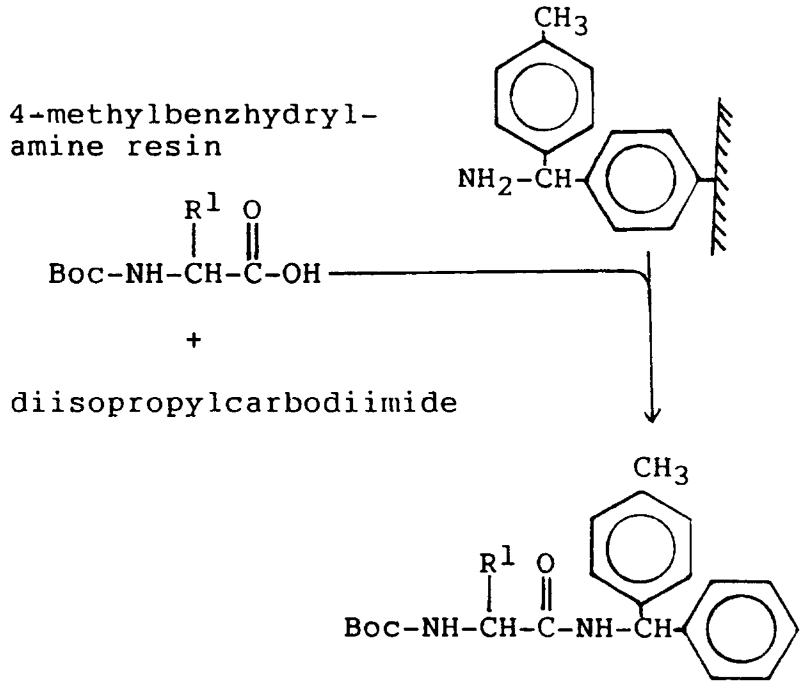


**Coupling the C-terminal Residue to 4-Methylbenzhydryl amine residue**

The peptides synthesized according to the above described procedure are highly reactive with antibodies to HTLV-1 and can be used as a highly sensitive and specific immunoadsorbent for the detection of the antibodies against HTLV-1.

Tables I and II show the data obtained with sera from ATL patients using an ELISA method wherein the well plates are coated with a mixture of the peptides in a weight ratio of 1:1:1 (I:II:III) and deactivated HTLV-1. Table III compares the data obtained with sera from ATL patients using an ELISA method wherein the well plates are coated respectively with each of the three peptides as well as a mixture (1:1:1) thereof and disrupted HTLV-1. Table IV shows the data obtained with sera from ATL patients using an ELISA method wherein the well plates are coated with a mixture of peptides II, IV, V and VI, in a weight ratio of 1:0.25:1:1 (II:IV:V:VI). Table V shows the data obtained with sera from ATL patients utilizing an agglutination method wherein the red blood cells (RBC) are coated with a peptide VI-BSA conjugate.

Based on the high degree of sensitivity and specificity of the peptide compositions according to the present invention in the immunoreaction to antibodies to HTLV-1, it is believed that the peptide compositions may also be useful as a vaccine for ATL, and as immunogens for the development of both monoclonal and polyclonal antibodies to HTLV-1 in mammals, including humans. The peptide compositions when coupled to a protein or synthesized on a polymer carrier resin (e.g., an octameric lysine resin) or when polymerised to homo or hetero dimers or higher oligomers by cysteine oxidation, induced disulfide cross linking, or when polymerized to homo or hetero dimers or higher oligomers by use of homo or hetero functional multivalent cross linking reagents, can be introduced to normal subjects to stimulate production of antibodies to HTLV-1, and provide protection against infection by HTLV-1 in healthy mammals. Since the peptide composition accordings to the present invention are not derived biochemically from the virus, there is no danger of exposing the normal subjects who are to be vaccinated to the disease.

The advantages of using the peptides according to the present invention are many.

The peptides are chemically synthesized. This means that there is no involvement with the HTLV-1 virus at any time during the process of making the test reagent or the vaccine. During the preparation of the vaccine or the vaccination process, production workers or individuals in the health professions do not risk exposure to the HTLV-1 virus. Similarly, there is no risk of exposure to HTLV-I in the use of these peptides or the development of monoclonal or polyclonal antibodies to HTLV-I in mammals. Further, up to the final step of the test to detect antibodies to HTLV-I, where the test reagent is exposed to samples of sera or body fluid, there is no risk of exposure of the laboratory worker to the HTLV-I virus.

Another problem which is avoided by the process of the present invention is the possibility of false positive results caused by the presence of antigenic materials from host cells co-purified with the HTLV-1 viral preparation or E-Coli derived proteins co-purified with expressed viral fragments. Certain normal individuals have antibodies to E. Coli or human leukocyte antigens, e.g. HLA, which are cross reactive with the antigenic materials from host cells. Sera samples from these normal individuals even though they have not been exposed to HTLV-1, may show a positive response in the ELISA or IRMA tests.

A diagnosis that a person may be infected with HTLV-1 based on this type of false positive response can bring severe anxiety to the person and his/her family. All of these problems can be avoided by using the peptide composition of the present invention as the test reagents.

Further, with appropriate amino acid analogue substitutions, it is expected that various peptide analogues based on the prescribed amino acid sequence can be synthesized with properties giving rise to lower background readings or better adsorption capacity to solid phases useful for HTLV-1 antibodies screening assays.

Moreover, because the peptide compositions of the present invention are synthetically prepared, the quality can be controlled and as a result, reproducibility of the test results can be assured. Also, since very small amounts of peptides are required for each test procedure, and because the expense of preparing the peptides is relatively low, the cost of screening body fluids for antibodies to HTLV-1, and diagnosis of ATL and the preparation of a vaccine is relatively low.

The peptides prepared in accordance with the present invention can be used to detect HTLV-1 infection and diagnose ATL by using it as the test reagent in an enzyme-linked immunoadsorbent assay (ELISA), an enzyme immunodot assay, a hemagglutination assay, a radioimmunoradiometric assay (IRMA), or other well-known immunoassays. The preferred method is ELISA. The ELISA technique is exemplified in Example 1, the IRMA technique is exemplified in Example 3, and the hemagglutination assay in Examples 4 and 5.

It is to be noted that in the following methods, 0.25% by weight of glutaraldehyde may be added in the coating buffer to facilitate better peptide binding onto the plates or beads. Further, horseradish peroxidase conjugated mouse monoclonal anti-human IgG antibody may be used in place of horseradish peroxidase conjugated goat anti human IgG as the second antibody tracer.

The gelatin used in these processes can include calf skin gelatin, pig skin gelatin, fish gelatin or any known available gelatin proteins or be replaced with albumin proteins.

## EXAMPLE 1

### Detection of Antibodies to HTLV-1 by an Enzyme-Linked Immunoadsorbent Assay

Wells of 96-well plates were coated at 4°C overnight (or 3 hours at room temperature), with a mixture of three peptides prepared as described in a ratio by weight of I:II:III = 1:1:1, at 1.5 µg per well of the mixture in 100 ul 10mM $NaHCO_3$ buffer, pH 9.5. The wells were washed three times with phosphate buffered saline (PBS) and then incubated with 250 ul of 3% by weight of gelatin in PBS at 37°C for 1 hour to block non-specific protein binding sites, followed by three more washes with PBS containing 0.05% by volume of Tween 20. The test sera (blood taken from a human patient or normal individual) were diluted with PBS containing 20% by volume normal goat serum, 1% by weight gelatin and 0.05% by volume Tween 20 at dilutions of 1:20 and 1:200, volume to volume, respectively. 200 µl of the diluted sera were added of each well and allowed to react for 1 hour at 37° C. The wells were then washed three times with 0.05% by volume Tween 20 in PBS in order to remove unbound antibodies. Horseradish peroxidase conjugated goat anti-human IgG was used as a second antibody tracer to bind with the HTLV-1 antibody-antigen complex formed in positive wells. 100 µl of peroxidase labeled goat anti human IgG at a dilution of 1:3000 in 1% by volume normal goat serum, 0.05% by volume Tween 20 in PBS was added to each well and incubated at 37°C for another 15 minutes.

The wells were washed five times with 0.05% by volume Tween 20 in PBS to remove unbound antibody and reacted with 100 µl of the substrate mixture containing 0.04% by weight orthophenylenediamine (OPD) and 0.012% by volume hydrogen peroxide in sodium citrate buffer, pH 5.0. This substrate mixture was used to detect the peroxidase label by forming a colored product. Reactions were stopped by the addition of 100 µl of 1.0M $H_2SO_4$ and the absorbance measured using an ELISA reader at 492nm (i.e. $A_{492}$). Assays were performed in duplicate with one dilution (1:20) of serum samples from normal individuals or from patients with diseases unrelated to HTLV-1 infection used as negative controls. Absorbance readings greater than the cutoff value of $A_{492}$ = 0.12, (about 3x the mean $A_{492}$ value of normal serum control), were taken as positive. The results are shown in Table I.

TABLE I

| Detection of Antibodies to HTLV-1 by ELISA* Using a Mixture of Three Peptides as Solid Phase Immunoadsorbent | | |
|---|---|---|
| Subject | No. Positive/No. Tested* | Percent Positive |
| 1. Patients with ATL | 102/102 | 100.0% |
| 2. Patients with AIDS/ARC or known to be infected with HTLV-III | 5/30 | 16.7% |
| 3. Patients with autoimmune diseases | 0/12 | 0 |
| 4. Normal Subjects | 0/10 | 0 |

*Assay was performed using sera at 1:20 (v/v) dilution with buffer. The cutoff value was defined as $A_{492}$ = 0.12, about three times (3X) the mean $A_{492}$ value of normal serum control.

Note:

Sera from patients with ATL (Lots I and II) were kindly provided by Dr. Kanji Miyamoto of the Japanese Okayama Red Cross, sera from patients with AIDS, ARC Primary Immunodeficiency, Leukemia/Lymphomas were kindly provided by Dr. S. Gupta at the University of California at Irvine; Dr. D. M. Knowles at the New York University, and Dr. F. D. Siegal at the Long Island Jewish Hospital. Sera from patients with autoimmune diseases including Rheumatoid Arthritus, systemic Lupus Erythematosus and allergies were kindly provided by Dr. N. Chiorazzi at the Rockefeller University Hospital, New York.

The results in Table I show that the ELISA test procedure according to the present invention with sera samples is very accurate and highly specific. Although, about 16.7% of the AIDS/ARC or HTLV-III (HIV) infected individuals were found also to be infected with HTLV-1, this is consistent with recent findings. These findings are alarming and effective measures are called for to prevent double infection by HTLV-1 and HIV (HTLV-III). No immunoreactivity was found in normal subjects or patients who were identified as not being infected with HTLV-1.

It is to be noted that in screening tests to exclude virus contaminated blood from blood banks, the criteria for defining positive reactions may be made more stringent if desired.

EXAMPLE 2

The procedure of Example 1 was repeated using the same sera samples as in Example 1 except that the well plates were precoated with 1 µg per well heat inactivated NP40 solubilized HTLV-1. The results are presented in Table II.

TABLE II

| Detection of Antibodies to HTLV-1 by ELISA Using Heat Inactivated NP40 Solubilized HTLV-1 as Solid Phase Immunoadsorbent | | |
|---|---|---|
| Subject | No. Positive/No. Tested* | Percent Positive |
| 1. Patients with ATL | 69/102 | 67.2 |
| 2. Patients with AIDS and known to be infected with HTLV-III | 2/12 | 16.7 |
| 3. Patients with autoimmune diseases | 0/12 | 0 |
| 4. Normal subjects | 0/12 | 0 |

*The cutoff value is defined as the highest $A_{492}$ of normal serum control.

In comparison with results obtained in Example 1, this method is much less accurate and specific and, therefore, less reliable. Furthermore, the cutoff value is selected using a much more liberal criteria.

EXAMPLE 3

Detection of Antibodies to HTLV-1 by an Immunoradiometric Assay (IRMA)

Wells of 96-well flexible-polyvinylchloride (PVC) plates are coated at 4°C overnight (or 3 hours at room temperature) with a mixture (1:1:1) of these three peptides, prepared as described, at 1.5 µg per well in 100 µl 10mM $NaHCO_3$ suffer, pH 9.5. The wells are washed three times with phosphate buffered saline (PBS) and then incubated with 250 µl of 3% by weight gelatin in PBS at 37°C for 1 hour to block non-specific protein binding sites, followed by three more washes with PBS containing 0.05% by volume Tween 20. The test sera (blood taken from a human patient or normal individual) are diluted with PBS containing 20% by volume normal goat serum, 1% by weight gelatin and 0.05% by volume Tween 20 at dilutions of 1:20 and 1:200 (volume to volume) respectively. 200 µl of the diluted sera are added to each cell and allowed to react for 1 hour at 37°C. The wells are then washed three times with 0.05% by volume Tween 20 in PBS in order to remove unbound antibodies. I-125 labeled affinity purified goat antihuman IgG is used as a second antibody tracer that binds with the antibody-antigen complex formed in positive wells. 100 µl of I-125 labeled goat antihuman IgG of 50,000-200,000 cpm in 1% by volume normal goat serum, 0.05% by volume Tween 20 in PBS is added to each well and incubated at 37°C for another hour.

The wells are washed five times with 0.05% by volume Tween 20 in PBS to remove unbound second antibody and dried. The wells are cut and counted by a gamma-scintillation counter. Assays are performed in duplicate with a 1:20 dilution volume to volume. Normal sera sample as negative controls are also tested simultaneously. Cpm readings greater than the average readings of normal sera samples + 4SD (standard deviation) are taken as positive.

EXAMPLE 4

Detection Of Antibodies To HTLV-1 By A Hemagglutination Assay Utilizing As The Solid Phase Immunoadsorbent Gelatin Particles, Erythrocytes Of Different Animal Species Or Latex Beads Coated With A Mixture Of Peptides

One ml thoroughly washed erythrocytes, gelatin particles, or polystyrene latex beads are coated with the peptide mixture at concentration in the range of 5 µg/ml to 1 mg/ml. The peptide mixture coated cells, particles or beads are then incubated with serially diluted serum samples in the wells of a 96-well U-shaped microplate. After being left at room temperature for about an hour, the agglutination patterns on the bottom are read, and the largest dilution showing a positive reaction is recorded.

This is a one-step assay which could be used for both qualitative and quantitative analysis of the presence of antibodies to HTLV-1 in specimens including sera or biofluids.

EXAMPLE 5

A third test kit for detecting HTLV-1 antibodies using the hemagglutination assay comprises a compartmented enclosure containing multiple 96-well U-shaped microplates and materials or hemagglutination assay including (1) a bottle of peptide mixture coated erythrocytes, gelatin particles or latex polystyrene beads; (2) normal human serum (as a negative control); and, (3) heat inactivated, NP40 solubilized seropositive ATL serum (as a positive control). The procedure described in Example 4 is to be followed.

EXAMPLE 6

A diagnostic test kit for HTLV-1 antibodies detection can be constructed. The test kit comprises a compartmented enclosure containing multiple 96-well plates coated prior to use with 1.5 µg per well of the peptide mixture (1:1:1) of the present invention in 100 µl pH 9.5 10mM $NaHCO_3$ buffer. The kit further comprises materials for enzyme detection in separate sealed containers consisting of: 1) normal human serum (as negative control); 2) heat inactivated, NP40 solubilized HTLV-1 seropositive ATL serum (as positive control); 3) normal goat serum; 4) peroxidase labeled-goat antihuman IgG; and 5) a color change indicator consisting of orthophenylenediamine (OPD) and hydrogen peroxide in phosphate citrate buffer. The procedure described in Example 1 is to be followed.

In this test kit, 96-well plates, precoated with the peptide of the present invention, can be replaced by polystyrene beads, or multiple mini-columns filled with controlled pore size glass beads, or nitrocellulose paper strip precoated with the peptides of the present invention for use as the solid phase immunoadsorbent.

EXAMPLE 7

A second test kit for detecting antibodies using the immunoradiometric assay (IRMA) comprises a compartmented enclosure containing multiple 96-well bendable polyvinylchloride (PVC) plates precoated with the peptide mixture (1:1:1) according to the present invention at a concentration of 1.5 µg per well of the peptide mixture in 100 µl of pH 9.5 10mM $NaHCO_3$ buffer and materials for radioiommunoassay including: 1) normal human serum (as negative control); 2) heat inactivated, NP40 solubilized seropositive ATL serum (as positive control); 3) normal goat serum; and, 4) I-125 labeled goated anti human IgG. The procedure described in Example 3 is to be followed.

In this test kit, 96- well PVC plates precoated with the peptides of the present invention can be replaced by polystyrene beads precoated with the peptide of the present invention for use as the solid phase immunoadsorbent.

EXAMPLE 8

An experiment was conducted to compare ATL HTLV-1 antibody results using individual peptides and a mixture (1:1:1) thereof in the procedure of Example 1 and heat inactivated, NP40 solubilized HTLV-1 according to Saxinger et al. Sera from ATL patients or from HTLV-1 infected, asymptomatic individuals were diluted 1:20. Duplicates of each diluted sera sample were tested against the peptides according to the present invention and cultured HTLV-1 according to Saxinger et al. Normal human serum and heat

inactivated HTLV-1 seropositive ATL serum were used as controls. The results are shown in Table III.

TABLE III

COMPARISON OF $A_{492}$/CUT OFF RATIO FOR 102 HTLV-1 POSITIVE SERA USING PEPTIDES I, II, III, MIXTURE THEREOF AND DISRUPTED HTLV-I AS SOLID PHASE IMMUNOADSORBENT

| LOT NO. | SAMPLE NO. | DISRUPTED HTLV-1 | PEPTIDE I | PEPTIDE II | PEPTIDE III | 1:1:1 I+II+III |
|---|---|---|---|---|---|---|
| I | 1 | 1.49 | 0.14 | 7.53 | 0.72 | 3.77 |
| | 2 | 1.88 | 12.17 | 30.72 | 4.78 | 36.23 |
| | 3 | 1.61 | 0.72 | 2.90 | 1.30 | 4.35 |
| | 4 | 1.54 | 4.78 | 28.40 | 5.94 | 37.54 |
| | 5 | 1.91 | 6.38 | 33.04 | 30.14 | 37.97 |
| | 6 | 1.70 | 13.19 | 1.30 | 1.45 | 1.59 |
| | 7 | 1.85 | 0.14 | 30.14 | 28.11 | 37.10 |
| | 8 | 1.84 | 11.30 | 6.52 | 2.02 | 10.14 |
| | 9 | 1.16 | 1.16 | 3.77 | 1.01 | 4.35 |
| | 10 | 1.68 | 1.16 | 6.96 | 1.16 | 8.99 |
| | 11 | 1.67 | 20.43 | 2.17 | 1.59 | 2.75 |
| | 12 | 2.37 | 18.70 | 12.60 | 3.62 | 18.40 |
| | 13 | 1.79 | 16.20 | 30.00 | 3.48 | 37.10 |
| | 14 | 2.11 | 13.30 | 3.33 | 12.75 | 17.68 |
| | 15 | 1.55 | 3.91 | 6.23 | 1.16 | 3.91 |
| | 16 | 1.21 | 2.32 | 2.03 | 0.72 | 1.74 |
| | 17 | 1.49 | 1.74 | 3.48 | 1.16 | 4.06 |
| | 18 | 1.49 | 7.39 | 2.90 | 1.74 | 2.32 |
| | 19 | 1.48 | 26.30 | 2.90 | 2.61 | 20.72 |
| | 20 | 1.27 | 1.88 | 6.52 | 1.88 | 7.25 |
| II | 1 | 0.00 | 0.14 | 2.03 | 1.30 | 3.19 |
| | 2 | 0.91 | 14.05 | 8.99 | 12.17 | 24.06 |
| | 3 | 1.88 | 0.00 | 3.62 | 1.59 | 1.59 |

| LOT NO. | SAMPLE NO. | DISRUPTED HTLV-1 | PEPTIDE I | II | III | 1:1:1 I+II+III |
|---|---|---|---|---|---|---|
| II | 4 | 0.67 | 20.14 | 5.94 | 10.00 | 12.46 |
| | 5 | 2.00 | 928.99 | 10.50 | 2.61 | 6.23 |
| | 6 | 1.69 | 0.00 | 8.84 | 1.59 | 9.13 |
| | 7 | 1.10 | 17.40 | 16.23 | 23.77 | 42.03 |
| | 8 | 1.03 | 2.61 | 14.78 | 17.10 | 11.88 |
| | 9 | 1.21 | 0.72 | 1.59 | 1.59 | 1.59 |
| | 10 | 0.85 | 2.61 | 2.03 | 2.17 | 3.48 |
| | 11 | 0.94 | 5.36 | 17.83 | 28.40 | 24.93 |
| | 12 | 1.25 | 3.48 | 21.30 | 13.04 | 30.87 |
| | 13 | 1.52 | 24.63 | 26.09 | 3.04 | 30.87 |
| | 14 | 1.91 | 7.10 | 13.38 | 31.74 | 36.81 |
| | 15 | 0.42 | 1.30 | 6.38 | 3.19 | 8.70 |
| | 16 | 1.25 | 0.00 | 4.64 | 3.19 | 6.96 |
| | 17 | 1.40 | 0.87 | 20.72 | 11.16 | 20.58 |
| | 18 | 0.015 | 0.00 | 1.30 | 1.74 | 1.59 |
| | 19 | 1.81 | 4.35 | 34.06 | 34.49 | 35.80 |
| | 20 | 1.37 | 0.00 | 4.78 | 1.16 | 7.10 |
| | 21 | 1.79 | 0.14 | 7.54 | 1.45 | 3.91 |
| | 22 | 1.26 | 0.14 | 2.90 | 1.88 | 3.33 |
| | 23 | 0.90 | 2.75 | 7.10 | 6.67 | 9.71 |
| | 24 | 0.73 | 0.29 | 1.74 | 2.32 | 2.32 |
| | 25 | 0.96 | 0.00 | 3.33 | 1.30 | 3.91 |
| | 26 | 1.34 | 0.00 | 6.52 | 2.03 | 4.20 |
| | 27 | 1.55 | 11.74 | 22.17 | 31.16 | 36.67 |
| | 28 | 2.03 | 21.74 | 33.33 | 32.46 | 38.41 |
| | 29 | 1.49 | 27.97 | 25.94 | 8.26 | 35.80 |
| | 30 | 1.69 | 3.48 | 3.04 | 1.45 | 3.19 |
| | 31 | 1.55 | 5.94 | 8.99 | 3.33 | 30.14 |

| LOT NO. | SAMPLE NO. | DISRUPTED HTLV-1 | PEPTIDE I | II | III | 1:1:1 I+II+III |
|---|---|---|---|---|---|---|
| II | 32 | 1.58 | 13.04 | 17.10 | 1.88 | 13.19 |
| | 33 | 1.43 | 6.23 | 16.67 | 1.88 | 17.97 |
| | 34 | 1.33 | 0.00 | 2.46 | 2.57 | 3.04 |
| | 35 | 1.60 | 3.91 | 7.39 | 14.35 | 13.04 |
| | 36 | 1.42 | 0.00 | 10.58 | 2.61 | 25.80 |
| | 37 | 1.22 | 0.43 | 1.88 | 1.74 | 2.03 |
| | 38 | 0.70 | 0.00 | 1.88 | 1.74 | 4.35 |
| | 39 | 1.49 | 10.29 | 16.23 | 1.74 | 26.23 |
| | 40 | 1.94 | 10.58 | 14.64 | 14.78 | 24.05 |
| | 41 | 1.73 | 15.36 | 20.29 | 14.35 | 35.65 |
| | 42 | 1.54 | 0.00 | 1.16 | 1.16 | 1.45 |
| | 43 | 1.70 | 0.00 | 1.59 | 1.74 | 1.88 |
| | 44 | 0.49 | 0.00 | 2.32 | 2.32 | 2.90 |
| | 45 | 0.52 | 0.00 | 1.74 | 1.88 | 2.03 |
| | 46 | 0.96 | 0.72 | 2.32 | 1.59 | 7.10 |
| | 47 | 1.79 | 28.99 | 35.94 | 24.50 | 37.68 |
| | 48 | 1.18 | 0.58 | 3.33 | 2.03 | 2.61 |
| | 49 | 1.72 | 8.40 | 17.25 | 2.18 | 15.79 |
| | 50 | 1.10 | 0.00 | 1.45 | 1.74 | 2.32 |
| | 51 | 1.63 | 8.41 | 15.94 | 1.45 | 19.57 |
| | 52 | 1.40 | 0.43 | 1.74 | 1.88 | 2.75 |
| | 53 | 1.10 | 0.00 | 3.04 | 2.46 | 3.33 |
| | 54 | 1.31 | 1.74 | 5.80 | 1.16 | 8.26 |
| | 55 | 0.46 | 0.72 | 2.60 | 2.02 | 3.3 |
| | 56 | 0.60 | 3.19 | 4.64 | 2.17 | 5.80 |
| | 57 | 0.40 | 0.87 | 3.48 | 2.02 | 33.33 |
| | 58 | 0.72 | 9.86 | 14.20 | 1.74 | 25.07 |
| | 59 | 0.46 | 0.00 | 2.90 | 0.72 | 4.20 |

| | | | PEPTIDE | | | |
|---|---|---|---|---|---|---|
| | SAMPLE | DISRUPTED | | | | 1:I:I |
| LOT NO. | NO. | HTLV-1 | I | II | III | I+II+III |
| II | 60 | 0.49 | 0.00 | 1.74 | 1.74 | 4.20 |
| | 61 | 0.48 | 7.83 | 12.6 | 3.04 | 12.17 |
| | 62 | 0.39 | 0.29 | 1.88 | 1.45 | 3.77 |
| | 63 | 0.34 | 2.17 | 6.09 | 3.49 | 8.55 |
| | 64 | 0.40 | 0.14 | 1.88 | 1.59 | 3.48 |
| | 65 | 0.37 | 17.2 | 18.69 | 11.30 | 35.65 |
| | 66 | 0.10 | 0.87 | 1.45 | 1.16 | 1.45 |
| | 67 | 1.68 | 0.58 | 8.99 | 1.88 | 14.20 |
| | 68 | 1.46 | 0.00 | 1.88 | 1.74 | 2.32 |
| | 69 | 0.61 | 1.59 | 9.13 | 2.46 | 13.76 |
| | 70 | 0.76 | 11.01 | 14.92 | 9.69 | 32.61 |
| | 71 | 1.54 | 0.43 | 1.56 | 2.03 | 3.04 |
| | 72 | 0.63 | 0.43 | 2.17 | 1.88 | 2.60 |
| | 73 | 2.03 | 23.77 | 36.96 | 20.29 | 28.89 |
| | 74 | 0.72 | 0.00 | 1.16 | 10.14 | 1.45 |
| | 75 | 0.54 | 0.00 | 1.45 | 1.30 | 2.46 |
| | 76 | 1.19 | 0.29 | 2.17 | 1.74 | 2.03 |
| | 77 | 2.34 | 0.00 | 8.41 | 1.88 | 4.06 |
| | 78 | 1.64 | 3.62 | 6.67 | 6.96 | 11.30 |
| | 79 | 1.70 | 6.96 | 9.57 | 1.59 | 5.94 |
| | 80 | 1.74 | 7.10 | 14.2 | 2.03 | 11.01 |
| | 81 | 1.57 | 10.5 | 26.1 | 12.0 | 30.14 |
| | 82 | 0.86 | 1.59 | 3.33 | 1.74 | 4.92 |

The results in Table III show that the method is highly sensitive and specific. The ratio of $A_{492}$: Cutoff values achieved using the peptide composition of the present invention against ATL sera samples at the same dilution is often much higher than that achieved using deactivated HTLV-1 against identical sera samples at identical dilutions. This is particularly true when a mixture (1:1:1) by weight of the peptides was used as the immuno-adsorbent. The data also show the peptide composition in the form of a mixture is highly accurate and no false negative results were obtained.

EXAMPLE 9

Detection of Antibodies to HTLV-1 by an Enzyme-Linked Immunoadsorbent Assay

Wells of 96-well plates were coated at 4°C overnight (or for 3 hours at room temperature or for 1 hour at 37°C), with a mixture of four peptides prepared as described in a ratio by weight of II:IV:V:VI = 1:0.25:1:1 at 3.25 µg per well of the mixture in 100 µl 10mM $NaHCO_3$ buffer, pH 9.5. The wells were washed three times with phosphate buffered saline (PBS) and then incubated with 250 µl of 3% by weight of gelatin in PBS at 37°C for 1 hour to block non-specific protein binding sites, followed by three more washes with PBS containing 0.05% by volume of Tween 20. The test sera (blood taken from human patients or normal individuals) were diluted with PBS containing 20% by volume normal goat serum, 1% by

weight gelatin and 0.05% by volume Tween 20 at dilutions of 1:20 and 1:200, volume to volume, respectively. 200 µl of the diluted sera were added of each well and allowed to react for 1 hour at 37°C. The wells were then washed three times with 0.05% by volume Tween 20 in PBS in order to remove unbound antibodies. Horseradish peroxidase conjugated goat anti-human IgG was used as a second antibody tracer to bind with the HTLV-1 antibody-antigen complex formed in positive wells. 100 µl of peroxidase labeled goat anti human IgG at a dilution of 1:3000 in 1% by volume normal goat serum, 0.05% by volume Tween 20 in PBS was added to each well and incubated at 37°C for another 15 minutes.

The wells were washed five times with 0.05% by volume Tween 20 in PBS to remove unbound antibody and reacted with 100 µl of the substrate mixture containing 0.04% by weight orthophenylenediamine (OPD) and 0.012% by volume hydrogen peroxide in sodium citrate buffer, pH 5.0. This substrate mixture was used to detect the peroxidase label by forming a colored product. Reactions were stopped by the addition of 100 µl of 1.0M $H_2SO_4$ and the absorbance measured using an ELISA reader at 492nm (i.e. $A_{492}$). Assays were performed in duplicate with one dilution (1:20) of serum samples from normal individuals or from patients with diseases unrelated to HTLV-1 infection used as negative controls. Absorbance readings greater than the cutoff value of $A_{492}$ = 0.17 [$A_{492}$ value for normal control + 0.1 ($A_{492}$ value for a reactive control)], were taken as positive. The results are shown in Table IV and Figure 4.

TABLE IV

| Detection of Antibodies to HTLV-1 by ELISA* Using a Mixture of Four Peptides as Solid Phase Immunoadsorbent | | |
|---|---|---|
| Subject | No. Positive/No. Tested* | Percent Positive |
| 1. Patients (Lot 5) with ATL (HTLV-1 Western Blot Positive) | 94/94 | 100 |
| 2. Patients (Lot 5) with ATL (HTLV-1 Western Blot Negative) | 0/6 | 0 |
| 3. Patients with AIDS/ARC or known to be infected with HIV | 10/161 | 6 |
| 4. Normal Subjects | 0/200 | 0 |

*Assay was performed using sera at 1:20 (v/v) dilution with buffer.
Note:
Sera from patients with ATL were kindly provided by the Japanese Red Cross, sera from patients with AIDS, ARC Primary Immunodeficiency, Leukemia/Lymphomas were kindly provided by Dr. S. Gupta at the University of California at Irvine, Dr. D. M. Knowles at the New York University, and Dr. F. D. Siegal at the Long Island Jewish Hospital.

The results in Table IV show that the ELISA test procedure according to the present invention with sera samples is very accurate and highly specific. Although, about 16.7% of the AIDS/ARC or HTLV-III (HIV) infected individuals were found also to be infected with HTLV-1, this is consistent with recent findings. These findings are alarming and effective measures are called for to prevent double infection by HTLV-1 and HIV. No immunoreactivity was found in sera from normal subjects.

It is to be noted that in screening tests to exclude virus contaminated blood from blood banks, the criteria for defining positive reactions may be made more stringent if desired.

## Example 10

### Detection Of Antibodies to HTLV-1 By An Agglutination Assay

The presently claimed HTLV-1 peptides, synthesized according to the Merrifield solid phase method, were conjugated to bovine serum albumin (BSA) which had been derivatized with m-maleimidobenzoyl-N-hydroxysuccinimide ester (MBS), essentially as described by Fu-Tong Liu et al, in Biochemistry 18:690-697 (1979). To 0.32 ml. of a BSA solution (100 mg/ml in 0.01 M phosphate buffer, pH 7.0) at room temperature was added 0.013 ml of an MBS solution (0.025 mg/ml in dimethylformamide). [The amount of MBS added to the BSA solution can be varied according to the optimal molar ratio of BSA to MBS determined for a specific conjugate studied]. The mixture was stirred at room temperature for 1 hour, after which it was centrifuged to remove any precipitated albumin. The clarified mixture was then subjected to gel filtration on

Sephadex G-25 and the protein-containing fractions, as detected by their absorbance at 280 nm, were pooled and stored frozen at -70 °C until needed.

The peptides were dissolved in $H_2O$ at 10 mg/ml. A predetermined amount of each peptide solution was added dropwise to the previously activated BSA-MBS solution and stirred at room temperature for 3 hours. The final peptide-BSA conjugates were separated from other free peptides by gel filtration or extensive dialysis. The ratio of peptide to BSA was determined by SDS-PAGE according to conventional methods.

In one example, conjugated peptide VI-BSA was then adsorbed to double aldehyde fixed human O erythrocytes at pH 4.0. The peptide-conjugate coated erythrocytes were then treated with $NaBH_4$ to prevent non-specific protein binding. The peptide-conjugate coated erythrocytes were then washed with PBS and incubated with 5% normal human serum-PBS solution. These processed cells were then used in an agglutination assay for the detection of HTLV-1 antibodies in both serum and plasma specimens.

A total of 100 sera from patients with adult T cell leukemia were tested for antibodies to HTLV-1 by (1) an enzyme immunoassay (EIA) employing HTLV-1 viral lysate as the solid phase [Dupont's HTLV-1 ELISA]; (2) the Western Blot (WB) analysis; (3) the above-described HTLV-1 agglutination assay employing peptide VI-BSA conjugate as the solid phase.

The results are shown in Table V.

TABLE V

| Number of ATL Samples Tested | EIA | Results WB | HTLV-1 Agglutination Assay |
|---|---|---|---|
| 77 | + | + | 77 positive + |
| 2 | + | indeterm. | 2 negative* |
| 21 | - | - | 21 negative |

* The two specimens that tested negative with the HTLV-1 agglutination assay were found to have antibodies only to the p19 core protein of HTLV-1

Example 11

Simultaneous Detection Of Antibodies To HTLV-1 and HIV (1 and 2) By An Enzyme Immunoassay Employing A Mixture Of Seven Chemically Synthesized Peptides

A solution containing seven of the chemically synthesized peptides of the present invention was used to coat the wells of 96 well plates, according to the procedure of Example I. Three of the peptides were derived from the HTLV-1 peptide family [II, IV and VI]; three, from the HIV-1 peptide family [VII, VIII and IX]; and one, from the HIV-2 peptide family [X]. The peptides II:IV::VI:VII:VIII:IX:X were present at a ratio of 2:0.2:2:10:1:1:5 for a total concentration of 21.2 $\mu$g/ml. A total of 771 specimens from donors known to be HIV-1 positive (155 specimens); HIV-2 positive (10 specimens); HTLV-1 positive by Western Blot (92 specimens); HTLV-1 negative by Western Blot (4 specimens); patients with autoimmune diseases (AI, 36 specimens); and, from random blood donors (RBD, 474 specimens), were tested on the peptide-coated plates for their respective retroviral immunoreactivities.

Performance of this synthetic peptide-based retroviralcombo EIA (HTLV-1 and HIV-1 and 2) with these specimens is illustrated in Figure 5. The results clearly indicate the usefulness of these HTLV-1 peptides in conjunction with the HIV peptides for the detection of retroviral infections.

It is to be understood that the above examples are illustrative of the present invention and are not meant to limit the scope thereof.

**Claims**

1. A peptide composition having specific immunoreactivities to antibodies to HTLV-1 comprising a peptide selected from the group consisting of:
   GLDLLFWEQGGLCKALQEQC-Z (I)
   QNRRGLDLLFWEQGGLCKALQEQC-Z (II)
   NRRGLDLLFWEQGGLC-Z (III)

APPLLPHSNLDHILEPSIPWKSKLLTLVQLTLQS-Z (IV)
SSTPLLYPSLALPAPHLTLPFNWTHCFDPQIQAICSSPCH-Z (V)
CFDPQIQAIVSSPCHNSLILPPFSLSPVPTLGSRSRRA-Z (VI)

wherein Z is -OH or $NH_2$, analogues thereof wherein amino acids may be added to, deleted from or substituted in the sequence as long as the immunoreactivity of the peptide to antibodies to HTLV-1 is substantially preserved; and mixtures, conjugates and polymers thereof.

**2.** A peptide composition according to claim 1, comprising a mixture of peptides I, II, III, IV, V and VI wherein I:II:III:IV:V:VI is in a ratio of 0.01 - 20 µg : 0.01 - 20 µg : 0.01 - 20 µg : 0.01 - 20 µg : 0.01 - 20 µg : 0.01 - 20 µg.

**3.** A peptide composition according to claim 1, comprising a mixture of peptides IV, V and VI wherein IV:V:VI is in a ratio of 0.01 - 20 µg : 0.01 - 20 µg : 0.01 - 20 µg.

**4.** A peptide composition according to claim 1, comprising a mixture of peptides II, IV, V and VI wherein II:IV:V:VI is in a ratio of 1 µg : 0.25 µg : 1 µg : 1 µg.

**5.** An immunoassay method for the detection of antibodies to HTLV-1 and diagnosis of ATL conditions comprising:

A. coating a solid support with an effective amount of a peptide composition comprising a peptide selected from the group consisting of:

GLDLLFWEQGGLCKAEQEQC-Z (I)
QNRRGLDLLFWEQGGLCKALQEQC-Z (II)
NRRGLDLLFWEQGGLC-Z (III)
APPLLPHSNLDHILEPSIPWKSKLLTLVQLTLQS-Z (IV)
SSTPLLYPSLALPAPHLTLPFNWTHCFDPQIQAICSSPCH-Z (V)
CFDPQIQAIVSSPCHNSLILPPFSLSPVPTLGSRSRRA-Z (VI)

wherein Z is -OH or $NH_2$, analogues thereof wherein amino acids may be added to, deleted from or substituted in the sequence as long as the immunoreactivity of the peptide to antibodies to HTLV-1 is substantially preserved; and segments, mixtures, conjugates and polymers thereof as the antigen;

B. adding a test sera diluted with a buffer wherein the antibodies to HTLV-1 in the test sera form a peptide-antibody complex with the peptide composition;

C. incubating the mixture; and

D. detecting the presence of the peptide-antibody complex.

**6.** An immunoassay method according to claim 5 wherein the solid support is coated with a mixture of the peptide IV, V and VI, wherein IV:V:VI is in a ratio of 0.01 - 20 µg : 0.01 - 20 µg : 0.01 - 20 µg.

**7.** An immunoassay method according to claim 5 wherein the solid support is coated with a mixture of the peptide II, IV, V and VI, wherein II:IV:V:VI is in a ratio of 1 µg : 0.25 µg : 1 µg : 1 µg.

**8.** An immunoassay method according to one of claims 5 to 7 wherein step D comprises: introducing a second known antibody labelled with an enzyme and a substrate which reacts with the enzyme to form a colored product.

**9.** An immunoassay method according to one of claims 5 to 7 wherein step D comprises: introducing a second known antibody labelled with a radioactive element.

**10.** An immunoassay method according to one of claims 5 to 7 wherein the peptide-antibody complex is detectable as an agglutination pattern.

**11.** An immunoassay method according to claim 5 wherein the solid support is a strip coated with the peptide composition in a multidot, multi-line or multi-square array.

**12.** An immunoassay method according to claim 6 wherein the solid support is a strip coated with the peptide composition in a multidot, multi-line or multi-square array.

**13.** An immunoassay method according to claim 7 wherein the solid support is a strip coated with the peptide composition in a multidot, multi-line or multi-square array.

**14.** A test kit for the detection of antibodies to HTLV and the diagnosis of ATL comprising:
a. a solid support;
b. coated onto the solid support, an immunoadsorbent comprising a peptide composition according to claim 1;
c. a sample of normal serum as negative control;
d. a sample of serum containing antibodies to HTLV as positive control; and
e. a buffer for diluting the serum samples.

**15.** A peptide composition having specific immunoreactivities to antibodies to HTLV-1 and HIV comprising at least one peptide selected from the group consisting of:
GGLDLLFWEQGGLCKALQEQC-Z (I)
QNRRGLDLLFWEQGGLCKALQEQC-Z (II)
NRRGLDLLFWEQGGLC-Z (III)
APPLLPHSNLDHILEPSIPWKSKLLTLVQLTLQS-Z (IV)
SSTPLLYPSLALPAPHLTLPFNWTHCFDPQIQAICSSPCH-Z (V)
CFDPQIQAIVSSPCHNSLILPPFSLSPVPTLGSRSRRA-Z (VI)
wherein Z is -OH or $NH_2$, analogues thereof wherein amino acids may be added to, deleted from or substituted in the sequence as long as the immunoreactivity to antibodies to HTLV-1 derived is substantially preserved, mixtures, conjugates and polymers thereof; and at least one peptide selected from the group consisting of:
RILAVERYLKDQQLLGIWGCS-Z (VII)
IWGCSGKLICTTAVPWNAS-Z (VIII)
IVRMYSPTSIL-Z (IX)
DQARLNSWGCAFRQVC-Z (X)
wherein Z is -OH or $NH_2$, analogues thereof wherein amino acids may be added to, deleted from or substituted in the sequence as long as the immunoreactivity to antibodies to HIV is substantially preserved, mixtures, conjugates and polymers thereof.

**16.** A peptide composition according to claim 1, comprising a mixture of peptides I, II, III, IV, V, VI, VII, VIII, IX and X wherein I:II:III:IV:V:VI:VII:VIII:IX:X is in a ratio of 0.01 - 20 µg : 0.01 - 20 µg : 0.01 - 20 µg : 0.01 - 20 µg : 0.01 -20 µg : 0.01 - 20 µg : 0.01 - 20 µg : 0.01 - 20 µg : 0.01 - 20 µg : 0.01 - 20 µg.

**17.** A peptide composition according to claim 13, comprising a mixture of peptides II, IV, VI, VII, VIII, IX and X wherein II:IV:VI:VII:VIII:IX:X is in a ratio of 0.01 - 20 µg : 0.01 - 20 µg : 0.01 - 20 µg : 0.01 - 20 µg : 0.01 -20 µg : 0.01 - 20 µg : 0.01 - 20 µg.

**18.** A peptide composition according to claim 13, comprising a mixture of peptides II, IV, VI, VII, VIII, IX and X wherein II:IV:VI:VII:VIII:IX:X is in a ratio of 2 µg : 0.2 µg : 2 µg : 10 µg : 1 µg : 1 µg : 1.5 µg.

**19.** An immunoassay method for the simultaneous detection of antibodies of HTLV-1 and HIV comprising:
A. coating a solid support with an effective amount of a peptide composition comprising at least one peptide selected from the group consisting of:
GGLDLLFWEQGGLCKALQEQC-Z (I)
QNRRGLDLLFWEQGGLCKALQEQC-Z (II)
NRRGLDLLFWEQGGLC-Z (III)
APPLLPHSNLDHILEPSIPWKSKLLTLVQLTLQS-Z (IV)
SSTPLLYPSLALPAPHLTLPFNWTHCFDPQIQAICSSPCH-Z (V)
CFDPQIQAIVSSPCHNSLILPPFSLSPVPTLGSRSRRA-Z (VI)
wherein Z is -OH or $NH_2$, analogues thereof wherein amino acids may be added to, deleted from or substituted in the sequence as long as the immunoreactivity to antibodies to HTLV-1 is substantially preserved, mixtures, conjugates and polymers thereof as the antigen; and at least one peptide selected from the group consisting of:
RILAVERYLKDQQLLGIWGCS-Z (VII)
IWGCSGKLICTTAVPWNAS-Z (VIII)
IVRMYSPTSIL-Z (IX)

DQARLNSWGCAFRQVC-Z (X)

wherein Z is -OH or $NH_2$, analogues thereof wherein amino acids may be added to, deleted from or substituted in the sequence as long as the immunoreactivity to antibodies to HIV is substantially preserved, mixtures, conjugates and polymers thereof as an antigen;

B. adding a test sera diluted with a buffer wherein the antibodies to HTLV-1 and HIV in the test sera form a peptide-antibody complex with the peptide composition;

C. incubating the mixture; and

D. detecting the presence of the peptide-antibody complexes.

**20.** An immunoassay method according to claim 12 wherein the solid support is coated with a mixture of the peptides II, IV, VI, VII, VIII, IX and X wherein II:IV:VI:VII:VIII:IX:X is in a ratio of 0.01 - 20 µg : 0.01 - 20 µg : 0.01 - 20 µg : 0.01 - 20 µg : 0.01 - 20 µg : 0.01 - 20 µg : 0.01 - 20 µg.

**21.** An immunoassay method according to claim 12 wherein the solid support is coated with a mixture of the peptides II, IV, VI, VII, VIII, IX and X wherein II:IV:VI:VII:VIII:IX:X is in a ratio of 2 µg : 0.2 µg : 2 µg : 10 µg : 1 µg : 1 µg : 1,5 µg.

**Patentansprüche**

**1.** Peptidzusammensetzung mit spezifischer Immunoreaktivität für Antikörper gegen HTLV-1,
**dadurch gekennzeichnet,** daß
sie ein aus

GLDLLFWEQGGLCKALQEQC-Z (I)
QNRRGLDLLFWEQGGLCKALQEQC-Z (II)
NRRGLDLLFWEQGGLC-Z (III)
APPLLPHSNLDHILEPSIPWKSKLLTLVQLTLQS-Z (IV)
SSTPLLYPSLALPAPHLTLPFNWTHCFDPQIQAICSSPCH-Z (V)
CFDPQIQAIVSSPCHNSLILPPFSLSPVPTLGSRSRRA-Z (VI)

ausgewähltes Peptid enthält, in dem Z -OH oder $NH_2$, Analogons davon, in denen Aminosäuren in der Sequenz hinzugefügt, entfernt oder substituiert werden können, solange die Immunoreaktivität des Peptids für Antikörper gegen HTLV-1 im wesentlichen beibehalten wird, und deren Mischungen, Konjugate und Polymere.

**2.** Peptidzusammensetzung nach Anspruch 1,
**dadurch gekennzeichnet,** daß
sie eine Mischung aus den Peptiden I, II, II, IV, V und VI enthält, in der I:II:III:IV:V:VI in einem Verhältnis von 0,01-20 µg:0,01-20 µg:0,01-20 µg:0,01-20 µg:0,01-20 µg:0,01-20 µg ist.

**3.** Peptidzusammensetzung nach Anspruch 1,
**dadurch gekennzeichnet,** daß
sie eine Mischung aus den Peptiden IV, V und VI enthält, in der IV:V:VI in einem Verhältnis von 0,01-20 µg:0,01-20 µg:0,01-20 µg ist.

**4.** Peptidzusammensetzung nach Anspruch 1,
**dadurch gekennzeichnet,** daß
sie eine Mischung aus den Peptiden II, IV, V und VI enthält, in der II:IV:V:VI in einem Verhältnis von 1 µg:0,25 µg:1 µg:1 µg ist.

**5.** Immunoassay-Verfahren zur Bestimmung von Antikörpern gegen HTLV-1 und zur Diagnose von ATL-Bedingungen,
**gekennzeichnet durch**

A. Beschichten eines festen Trägers mit einer wirksamen Menge einer Peptidzusammensetzung enthaltend ein aus

GLDLLFWEQGGLCKALQEQC-Z (I)
QNRRGLDLLFWEQGGLCKALQEQC-Z (II)
NRRGLDLLFWEQGGLC-Z (III)
APPLLPHSNLDHILEPSIPWKSKLLTLVQLTLQS-Z (IV)

SSTPLLYPSLALPAPHLTLPFNWTHCFDPQIQAICSSPCH-Z (V)

CFDPQIQAIVSSPCHNSLILPPFSLSPVPTLGSRSRRA-Z (VI)

ausgewähltes Peptid, in dem Z -OH oder $NH_2$ ist, deren Analogons, in denen Aminosäuren der Sequenz hinzugefügt, entfernt oder substituiert werden können, solange die Immunoreaktivität des Peptids für Antikörper gegen HTLV-1 im wesentlichen beibehalten wird, und deren Segmenten, Mischungen, Konjugaten und Polymeren als Antigen;

B. Hinzufügen eines mit einem Puffer verdünnten Testserums, in dem die Antikörper gegen HTLV-1 in dem Testserum einen Peptid-Antikörper-Komplex mit der Peptidzusammensetzung bilden,

C. Inkubieren der Mischung und

D. Bestimmen des Vorliegens des Peptid-Antikörper-Komplexes.

**6.** Immunoassay-Verfahren nach Anspruch 5,
**dadurch gekennzeichnet,** daß
der feste Träger mit einer Mischung aus den Peptiden IV, V und VI beschichtet ist, in der IV:V:VI in einem Verhältnis von 0,01-20 µg:0,01-20 µg:0,01-20 µg ist.

**7.** Immunoassay-Verfahren nach Anspruch 5,
**dadurch gekennzeichnet,** daß
der feste Träger mit einer Mischung aus den Peptiden II, IV, V und VI beschichtet ist, in der II:IV:V:VI in einem Verhältnis von 1 µg:0,25 µg:1 µg:1 µg ist.

**8.** Immunoassay-Verfahren nach einem der Ansprüche 5 bis 7,
**dadurch gekennzeichnet,** daß
der Schritt D das Einführen eines zweiten mit einem Enzym markierten bekannten Antikörpers und eines Substrates umfaßt, welches mit dem Enzym unter Bildung eines gefärbten Produktes reagiert.

**9.** Immunoassay-Verfahren nach einem der Ansprüche 5 bis 7,
**dadurch gekennzeichnet,** daß
der Schritt D das Einführen eines zweiten mit einem radioaktiven Element markierten bekannten Antikörpers umfaßt.

**10.** Immunoassay-Verfahren nach einem der Ansprüche 5 bis 7,
**dadurch gekennzeichnet,** daß
der Peptid-Antikörper-Komplex als ein Agglutinationsmuster bestimmbar ist.

**11.** Immunoassay-Verfahren nach Anspruch 5,
**dadurch gekennzeichnet,** daß
der feste Träger ein mit der Peptidzusammensetzung beschichteter Streifen in einem Multidot-, Multi-Line- oder Multi-Square-Array ist.

**12.** Immunoassay-Verfahren nach Anspruch 6,
**dadurch gekennzeichnet,** daß
der feste Träger ein mit der Peptidzusammensetzung beschichteter Streifen in einem Multidot-, Multi-Line- oder Multi-Square-Array ist.

**13.** Immunoassay-Verfahren nach Anspruch 7,
**dadurch gekennzeichnet,** daß
der feste Träger ein mit der Peptidzusammensetzung beschichteter Streifen in einem Multidot-, Multi-Line- oder Multi-Square-Array ist.

**14.** Test-Kit zur Bestimmung von Antikörpern gegen HTLV und zur Diagnose von ATL,
**dadurch gekennzeichne,** daß es

a. einen festen Träger,

b. ein eine Peptidzusammensetzung nach Anspruch 1 enthaltendes Immunoadsorbens, das auf den festen Träger aufgebracht ist,

c. eine Probe eines Normalserums als Negativkontrolle,

d. eine Probe eines Antikörpers gegen HTLV als Positivkontrolle enthaltenden Serums und

e. einen Puffer zum Verdünnen der Serumproben
aufweist.

**15.** Peptidzusammensetzung mit spezifischer Immunoreaktivität für Antikörper gegen HTLV-1 und HIV, **dadurch gekennzeichnet,** daß
sie zumindest eines aus
GGLDLLFWEQGGLCKALQEQC-Z (I)
QNRRGLDLLFWEQGGLCKALQEQC-Z (II)
NRRGLDLLFWEQGGLC-Z (III)
APPLLPHSNLDHILEPSIPWKSKLLTLVQLTLQS-Z (IV)
SSTPLLYPSLALPAPHLTLPFNWTHCFDPQIQAICSSPCH-Z (V)
CFDPQIQAIVSSPCHNSLILPPFSLSPVPTLGSRSRRA-Z (VI)
ausgewählten Peptids, in dem Z -OH oder $NH_2$ ist, deren Analogons, in denen Aminosäuren in der Sequenz hinzugefügt, entfernt oder substituiert werden können, solange die Immunoreaktivität gegen sich von HTLV-1 herleitenden Antikörpern im wesentlichen beibehalten wird, und deren Mischungen, Konjugaten und Polymeren, und zumindest eines aus
RILAVERYLKDQQLLGIWGCS-Z (VII)
IWGCSGKLICTTAVPWNAS-Z (VIII)
IVRMYSPTSIL-Z (IX)
DQARLNSWGCAFRQVC-Z (X)
ausgewählten Peptids enthält, in dem Z -OH oder $NH_2$ ist, deren Analogons, in denen Aminosäuren in der Sequenz hinzugefügt, entfernt oder substituiert werden können, solange die Immunoreaktivität für Antikörper gegen HIV im wesentlichen beibehalten wird, deren Mischungen, Konjugaten und Polymeren.

**16.** Peptidzusammensetzung nach Anspruch 1, **dadurch gekennzeichnet,** daß
sie eine Mischung von Peptiden I, II, III, IV, V, VI, VII, VIII, IX und X enthält, in der I:II:III:IV:V:VI:VII:VIII:IX:X in einem Verhältnis von 0,01-20 µg:0,01-20 µg:0,01-20 µg:0,01-20µg:0,01-20µg:0,01-20µg:0,01-20µg:0,01-20 µg:0,01-20 µg:0,01-20 µg enthalten ist.

**17.** Peptidzusammensetzung nach Anspruch 13, **dadurch gekennzeichnet,** daß
sie eine Mischung von Peptiden II, IV, VI, VII, VIII, IX und X enthält, in der II:IV:VI:VII:VIII:IX:X in einem Verhältnis von 0,01-20 µg:0,01-20 µg:0,01-20 µg:0,01-20 µg:0,01-20 µg:0,01-20 µg:0,01-20 µg enthalten ist.

**18.** Peptidzusammensetzung nach Anspruch 13, **dadurch gekennzeichnet,** daß
sie eine Mischung von Peptiden II, IV, VI, VII, VIII, IX und X enthält, in der II:IV:VI:VII:VIII:IX:X in einem Verhältnis von 2 µg:0,2 µg:2 µg:10 µg:1 µg:1 µg:1,5 µg enthalten ist.

**19.** Immunoassay-Verfahren zur gleichzeitigen Bestimmung von Antikörpern gegen HTLV-1 und HIV, **gekennzeichnet** durch:

A. Beschichten eines festen Trägers mit einer wirksamen Menge einer Peptidzusammensetzung, enthaltend zumindest ein aus
GGLDLLFWEQGGLCKALQEQC-Z (I)
QNRRGLDLLFWEQGGLCKALQEQC-Z (II)
NRRGLDLLFWEQGGLC-Z (III)
APPLLPHSNLDHILEPSIPWKSKLLTLVQLTLQS-Z (IV)
SSTPLLYPSLALPAPHLTLPFNWTHCFDPQIQAICSSPCH-Z (V)
CFDPQIQAIVSSPCHNSLILPPFSLSPVPTLGSRSRRA-Z (VI)
ausgewähltes Peptid, in dem Z -OH oder $NH_2$ ist, deren Analogons, in denen die Aminosäuren in der Sequenz hinzugefügt, entfernt oder substituiert werden können, solange die Immunoreaktivität für Antikörper gegen HTLV-1 im wesentlichen beibehalten wird, deren Mischungen, Konjugate und Polymeren als Antigen; und zumindest ein aus
RILAVERYLKDQQLLGIWGCS-Z (VII)

IWGCSGKLICTTAVPWNAS-Z (VIII)
IVRMYSPTSIL-Z (IX)
DQARLNSWGCAFRQVC-Z (X)
ausgewähltes Peptid, in dem Z -OH oder $NH_2$ ist, deren Analogons, in denen die Aminosäuren in der Sequenz hinzugefügt, entfernt oder substituiert werden können, solange die Immunoreaktivität für Antikörper gegen HIV im wesentlichen beibehalten wird, deren Mischungen, Konjugate und Polymere als Antigen,
B. Hinzufügen eines mit einem Puffer verdünnten Testserums, in dem die Antikörper gegen HTLV-1 und HIV im Testserum einen Peptid-Antikörper-Komplex mit der Zusammensetzung bilden,
C. Inkubieren der Mischung und
D. Bestimmen des Vorliegens des Peptid-Antikörper-Komplexes.

**20.** Immunoassay-Verfahren nach Anspruch 12,
**dadurch gekennzeichnet,** daß
der feste Träger mit einer Mischung der Peptide II, IV, VI, VII, VIII, IX und X beschichtet ist, in denen II:IV:VI:VII:VIII:IX:X in einem Verhältnis von 0,01-20 µg:0,01-20µg:0,01-20 µg:0,01-20 µg:0,01-20 µg:0,01-20 µg:0,01-20 µg enthalten ist.

**21.** Immunoassay-Verfahren nach Anspruch 12,
**dadurch gekennzeichnet,** daß
der feste Träger mit einer Mischung der Peptide II, IV, VI, VII, VIII, IX und X beschichtet ist, in denen II:IV:VI:VII:VIII:IX:X in einem Verhältnis von 2µg:0,2 µg:2 µg:10 µg:1 µg:1 µg:1,5 µg enthalten ist.

**Revendications**

**1.** Un composé peptidique, présentant des immunoréactivités spécifiques aux anticorps anti - HTLV-1, comprenant un peptide choisi dans le groupe constitué par :
GLDLLFWEQGGLCKALQEQC-Z (I)
QNRRGLDLLFWEQGGLCKALQEQC-Z (II)
NRRGLDLLFWEQGGLC-Z (III)
APPLLPHSNLDHILEPSIPWKSKLLTLVQLTLQS-Z (IV)
SSTPLLYPSLALPAPHLTLPFNWTHCFDPQIQAICSSPCH-Z (V)
CFDPQIQAIVSSPCHNSLILPPFSLSPVPTLGSRSRRA-Z (VI)
où Z représente -OH ou $NH_2$, leurs analogues, dans lequel les acides aminés peuvent être ajoutés, supprimés ou substitués dans la séquence, tant que l'immunoréactivité du peptide aux anticorps anti - HTLV-1 est essentiellement préservée; et leurs mélanges, conjugués et polymères.

**2.** Un composé peptidique conforme à la revendication 1, comprenant un mélange des peptides I, II, III, IV, V et VI, où I:II:III:IV:V:VI est dans un rapport de 0,01 - 20 µg : 0,01 - 20 µg : 0,01 - 20 µg : 0,01 - 20 µg : 0,01 - 20 µg : 0,01 - 20 µg.

**3.** Un composé peptidique, conforme à la revendication 1, comprenant un mélange des peptides IV, V et VI, où IV:V:VI est dans un rapport de 0,01 - 20 µg : 0,01 - 20 µg : 0,01 - 20 µg.

**4.** Un composé peptidique, conforme à la revendication 1, comprenant un mélange des peptides II, IV, V et VI, où II:IV:V:VI est dans un rapport de 1 µg : 0,25 µg : 1 µg : 1 µg.

**5.** Une méthode d'essai immunologique pour la détection des anticorps anti - HTLV-1 et le diagnostic des conditions ATL comprenant les étapes suivantes :
A. enduire un support solide avec une quantité effective d'un composé peptidique, comprenant un peptide choisi dans le groupe constitué par :
GLDLLFWEQGGLCKALQEQC-Z (I)
QNRRGLDLLFWEQGGLCKALQEQC-Z (II)
NRRGLDLLFWEQGGLC-Z (III)
APPLLPHSNLDHILEPSIPWKSKLLTLVQLTLQS-Z (IV)
SSTPLLYPSLALPAPHLTLPFNWTHCFDPQIQAICSSPCH-Z (V)
CFDPQIQAIVSSPCHNSLILPPFSLSPVPTLGSRSRRA-Z (VI)
où Z représente -OH ou $NH_2$, leurs analogues, dans lequel les acides aminés peuvent être ajoutés,

supprimés ou substitués dans la séquence, tant que l'immunoréactivité du peptide aux anticorps anti - HTLV-1 est essentiellement préservée; et leurs segments, mélanges, conjugués et polymères, comme antigènes;

B. ajouter des sérums d'essai dilués avec un tampon, où les anticorps anti - HTLV-1 dans les sérums d'essai forment un complexe peptide - anticorps avec le composé peptidique;

C. incuber le mélange; et

D. détecter la présence du complexe peptide - anticorps.

**6.** Une méthode d'essai immunologique conforme à la revendication 5, où le support solide est enduit d'un mélange des peptides IV, V et VI, où IV:V:VI est dans un rapport de 0,01 - 20 µg : 0,01 - 20 µg : 0,01 - 20 µg.

**7.** Une méthode d'essai immunologique conforme à la revendication 5, où le support solide est enduit d'un mélange des peptides II, IV, V et VI, où II:IV:V:VI est dans un rapport de 1 µg : 0,25 µg : 1 µg : 1 µg.

**8.** Une méthode d'essai immunologique conforme à l'une des revendications 5 à 7, où l'étape D comprend : introduire un deuxième anticorps connu, marqué par un enzyme et un substrat, qui réagit avec l'enzyme pour former un produit coloré.

**9.** Une méthode d'essai immunologique conforme à lune des revendications 5 à 7, où l'étape D comprend : introduire un deuxième anticorps connu, marqué par un élément radioactif.

**10.** Une méthode d'essai immunologique conforme à l'une des revendications 5 à 7, où le complexe peptide - anticorps peut être détecté sous la forme d'un schéma d'agglutination.

**11.** Une méthode d'essai immunologique conforme à la revendication 5, où le support solide est une bandelette enduite du composé peptidique, dans une disposition multi-points, multi-lignes ou multi-carrés.

**12.** Une méthode d'essai immunologique conforme à la revendication 6, où le support solide est une bandelette enduite du composé peptidique, dans une disposition multi-points, multi-lignes ou multi-carres.

**13.** Une méthode d'essai immunologique conforme à la revendication 7, où le support solide est une bandelette enduite du composé peptidique, dans une disposition multi-points, multi-lignes ou multi-carrés.

**14.** Un kit d'essai pour la détection des anticorps anti - HTLV et le diagnostic de ATL comprenant :

a. un support solide;

b. un immunoadsorbant, enduit sur le support solide, et comprenant un composé peptidique conforme à la revendication 1;

c. un échantillon de sérum normal, servant de contrôle négatif;

d. un échantillon de sérum contenant des anticorps anti - HTLV servant de contrôle positif; et

e. un tampon pour diluer les échantillons de sérum.

**15.** Un composé peptidique, présentant des immunoréactivités spécifiques aux anticorps anti - HTLV-1 et anti - HIV, comprenant un peptide choisi dans le groupe constitué par :

GLDLLFWEQGGLCKALQEQC-Z (I)

QNRRGLDLLFWEQGGLCKALQEQC-Z (II)

NRRGLDLLFWEQGGLC-Z (III)

APPLLPHSNLDHILEPSIPWKSKLLTLVQLTLQS-Z (IV)

SSTPLLYPSLALPAPHLTLPFNWTHCFDPQIQAICSSPCH-Z (V)

CFDPQIQAIVSSPCHNSLILPPFSLSPVPTLGSRSRRA-Z (VI)

où Z représente -OH ou $NH_2$, leurs analogues, dans lequel les acides aminés peuvent être ajoutés, supprimés ou substitués dans la séquence, tant que l'immunoréactivité aux anticorps anti - HTLV-1 dérivés est essentiellement préservée; et leurs mélanges, conjugués et polymères; et au moins un peptide choisi dans le groupe constitué par :

RILAVERYLKDQQLLGIWGCS-Z (VII)
IWGCSGKLICTTAVPWNAS-Z (VII)
IVRMYSPTSIL-Z (IX)
DQARLNSWGCAFRQVC-Z (X)

où Z représente -OH ou $NH_2$, leurs analogues, dans lequel les acides aminés peuvent être ajoutés, supprimés ou substitués dans la séquence, tant que l'immunoréactivité aux anticorps anti - HIV est essentiellement préservée; et leurs mélanges, conjugués et polymères;

**16.** Un composé peptidique conforme à la revendication 1, comprenant un mélange des peptides I, II, III, IV, V, VI, VII, VIII, IX et X, où I:II:III:IV:V:VI:VII:VIII:IX:X est dans un rapport de 0,01 - 20 µg : 0,01 - 20 µg : 0,01 - 20 µg : 0,01 - 20 µg : 0,01 - 20 µg : 0,01 - 20 µg : 0,01 - 20 µg : 0,01 - 20 µg : 0,01 - 20 µg : 0,01 - 20 µg.

**17.** Un composé peptidique conforme à la revendication 13, comprenant un mélange des peptides II, IV, VI, VII, VIII, IX et X, où II:IV:VI:VII:VIII:IX:X est dans un rapport de 0,01 - 20 µg: 0,01 - 20 µg : 0,01 - 20 µg : 0,01 - 20 µg : 0,01 - 20 µg : 0,01 - 20 µg: 0,01 - 20 µg.

**18.** Un composé peptidique conforme à la revendication 13, comprenant un mélange des peptides II, IV, VI, VII, VIII, IX et X, où II:IV:VI:VII:VIII:IX:X est dans un rapport de 2 µg : 0,2 µg : 2 µg : 10 µg : 1 µg : 1 µg : 1,5 µg.

**19.** Une méthode d'essai immunologique pour la détection simultanée des anticorps anti - HTLV-1 et anti - HIV, comprenant les étapes suivantes :

A. enduire un support solide avec une quantité effective d'un composé peptidique, comprenant un peptide choisi dans le groupe constitué par :

GLDLLFWEQGGLCKALQEQC-Z (I)
QNRRGLDLLFWEQGGLCKALQEQC-Z (II)
NRRGLDLLFWEQGGLC-Z (III)
APPLLPHSNLDHILEPSIPWKSKLLTLVQLTLQS-Z (IV)
SSTPLLYPSLALPAPHLTLPFNWTHCFDPQIQAICSSPCH-Z (V)
CFDPQIQAIVSSPCHNSLILPPFSLSPVPTLGSRSRRA-Z (VI)

où Z représente -OH ou $NH_2$, leurs analogues, dans lequel les acides aminés peuvent être ajoutés, supprimés ou substitués dans la séquence, tant que l'immunoréactivité du peptide aux anticorps anti - HTLV-1 est essentiellement préservée; et leurs mélanges, conjugués et polymères, comme antigènes; et au moins un peptide choisi dans le groupe constitué par :

RILAVERYLKDQQLLGIWGCS-Z (VII)
IWGCSGKLICTTAVPWNAS-Z (VIII)
IVRMYSPTSIL-Z (IX)
DQARLNSWGCAFRQVC-Z (X)

où Z représente -OH ou $NH_2$, leurs analogues, dans lequel les acides aminés peuvent être ajoutés, supprimés ou substitués dans la séquence, tant que l'immunoréactivité aux anticorps anti - HIV est essentiellement préservée; et leurs mélanges, conjugués et polymères, comme antigènes;

B. ajouter des sérums d'essai dilués avec un tampon, où les anticorps anti - HTLV-1 et anti - HIV dans les sérums d'essai forment un complexe peptide - anticorps avec le composé peptidique ;

C. incuber le mélange; et

D. détecter la présence des complexes peptide - anticorps.

**20.** Une méthode d'essai immunologique conforme à la revendication 12, où le support solide est enduit d'un mélange des peptides II, IV, VI, VII, VIII, IX et X, où II:IV:VI:VII:VIII:IX:X est dans un rapport de 0,01 - 20 µg : 0,01 - 20 µg : 0,01 - 20 µg : 0,01 - 20 µg : 0,01 - 20 µg : 0,01 - 20 µg : 0,01 - 20 µg.

**21.** Une méthode d'essai immunologique conforme à la revendication 12, où le support solide est enduit d'un mélange des peptides II, IV, VI, VII, VIII, IX et X, où II:IV:VI:VII:VIII:IX:X est dans un rapport de 2 µg : 0,2 µg : 2 µg : 10 µg : 1 µg : 1 µg : 1,5 µg.

FIG.1-1

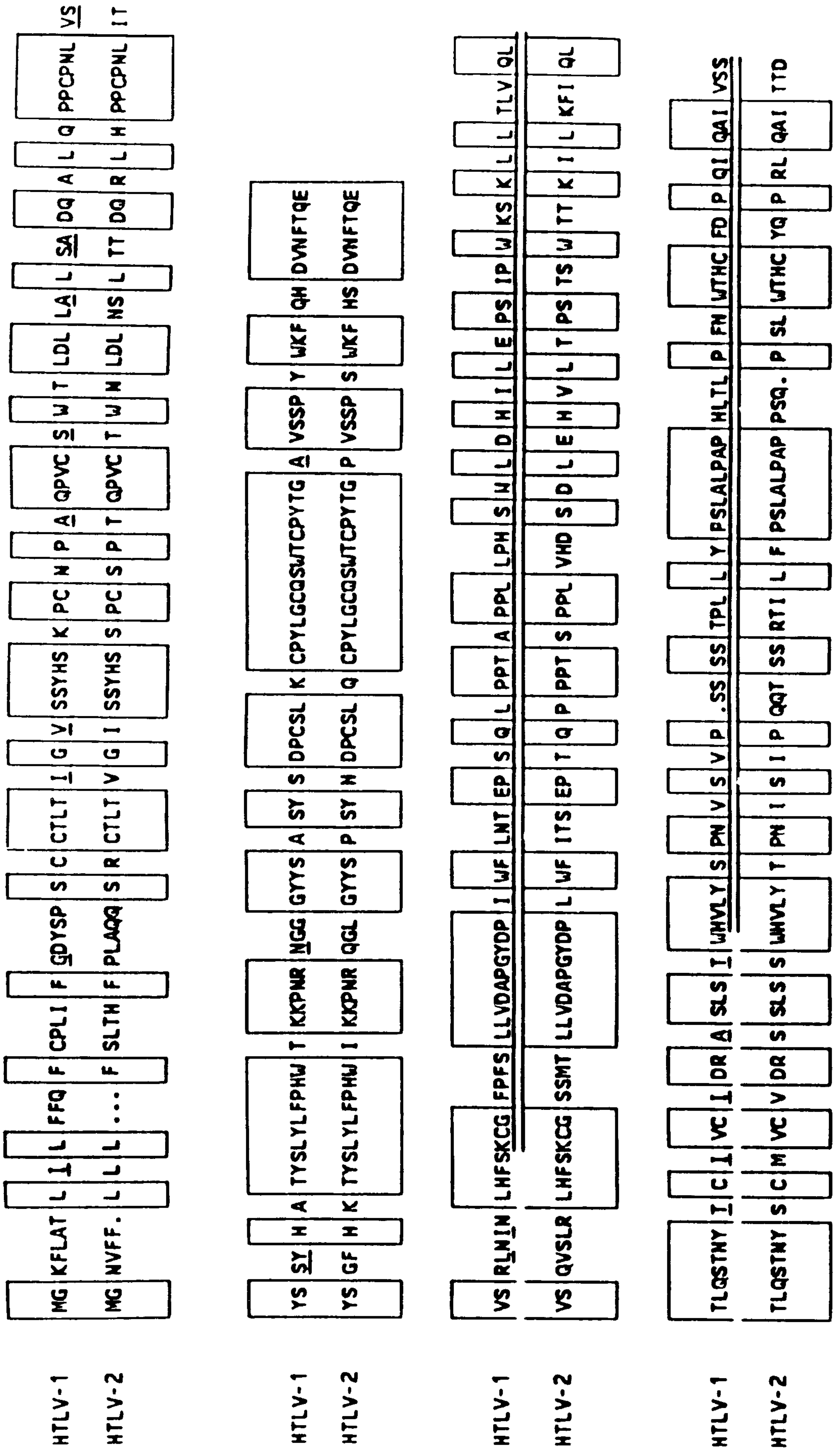

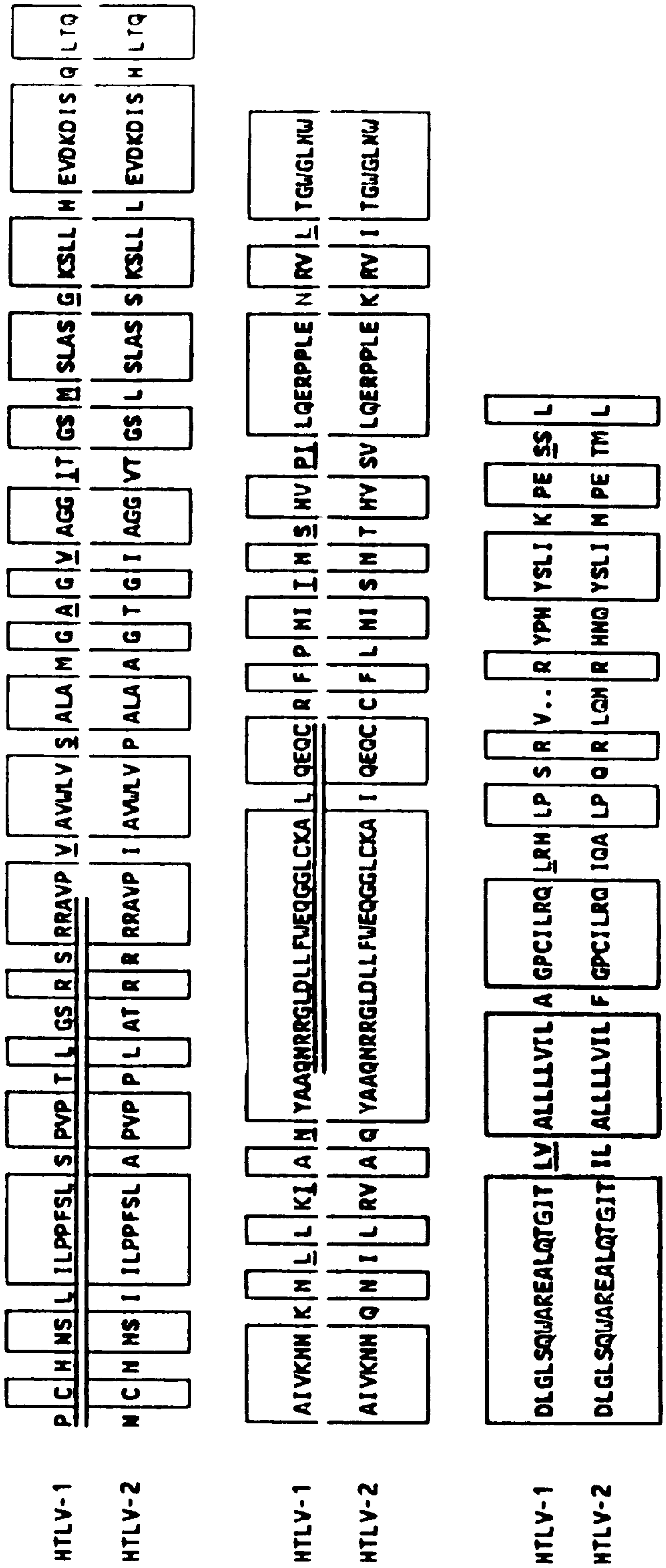
HTLV-1 P C H NS L ILPPFSL S PVP T L GS R S RRAVP V AVWLV S ALA M G A G V AGG IT GS M SLAS G KSLL H EVDKDIS Q LTQ
HTLV-2 N C N NS I ILPPFSL A PVP P L AT R R RRAVP I AVWLV P ALA A G T G I AGG VT GS L SLAS S KSLL L EVDKDIS H LTQ
HTLV-1 AIVKNH K N L L KI A N YAAQNRRGLDLLFWEQGGLCKA L QEQC R F P NI I N S HV PI LQERPPLE N RV L TGWGLNW
HTLV-2 AIVKNH Q N I L RV A Q YAAQNRRGLDLLFWEQGGLCKA I QEQC C F L NI S N T HV SV LQERPPLE K RV I TGWGLNW
HTLV-1 DLGLSQWAREALQTGIT LV ALLLLVIL A GPCILRQ LRH LP S R V.. R YPH YSLI K PE SS L
HTLV-2 DLGLSQWAREALQTGIT IL ALLLLVIL F GPCILRQ IQA LP Q R LQH R HNQ YSLI N PE TM L

FIG.1-2

EPITOPE I:

| Sequence | |
|---|---|
| FSLLVDAPGYDPIWFLNTEPSQLPPTAPPLLPHSNLDHILEPSIPWKSKLLTLVQLTLQS | HTLV-1 |
| MTLLVDAPGYDPLWFITSEPTQPPPTSPPLVHDSDLEHVLTPSTSWTTKILKFIQLTLQS | HTLV-2 |

| Sequence | Peptide No. | Relative Immunoreactivity |
|---|---|---|
| APPLLPHSNLDHILEPSIPWKSKLLTLVQLTLQS | IV | ++++ |
| FSLLVDAPGYDPIWFLMTEPSQLPPTAPPLLPHSN | XII | +/- |
| SPPLVHDSDLEHLVTPSTSWTTKILKFIQLTLQS | XIV(anal.*of IV) | ++++# |

EPITOPE II:

| Sequence | |
|---|---|
| HVLYSPNVSVPSSSSTPLLYPSLALPAPHLTLPFNWTHCFDPQIQAIVSSPCHNSLILPPFSLSPVPTLGSRSRRA | HTLV-1 |
| HVLVTPNISIPQTSSRTILFPSLALPAPPSQ-PSLWTHCYQPRLQAITTDNCNNSIILPPFSLAPVPPLATRRRRA | HTLV-2 |

| Sequence | Peptide No. | Relative Immunoreactivity |
|---|---|---|
| HVLYSPNVSVPSSSSTPLLYPSLALPAPHLTL | XIII | +/- |
| SSTPLLYPSLALPAPHLTLPFNWTHCFDPQIQAIVSSPCH | Vf | ++++ |
| LYPSLALPAPHLTLPFNWTHCFDPQIQAIVSSPCH | Ve | +++ |
| ALPAPHLTLPFNWTHCFDPQIQAIVSSPCH | Vd | ++++ |
| HLTLPFNWTHCFDPQIQAIVSSPCH | Vc | ++++ |
| FNWTHCFDPQIQAIVSSPCH | Vb | ++ |
| CFDPQIQAIVSSPCH | Va | ++ |
| CFDPQIQAIVSSPCHNSLILPPFSLSPVPTLGSRSRRA | VI | ++++ |
| QIQAIVSSPCHNSLILPPFSLSPVPTLGSRSRRA | VIe | +++ |
| IVSSPCHNSLILPPFSLSPVPTLGSRSRRA | VId | ++ |
| HNSLILPPFSLSPVPTLGSRSRRA | VIc | + |
| ILPPFSLSPVPTLGSRSRRA | VIb | +/- |
| SLSPVPTLGSRSRRA | VIa | +/- |
| ALPAPPSQ-PSLWTHCYQPRLQAITTDNCN | XIa(anal.*of Va-f) | +/- |
| LFPSLALPAPPSQ-PSLWTHCYQPRLQAITTDNCN | XIb | + |
| SSRTILFPSLALPAPPSQ-PSLWTHCYQPRLQAITTDNCN | XI | ++ |

FIG.2

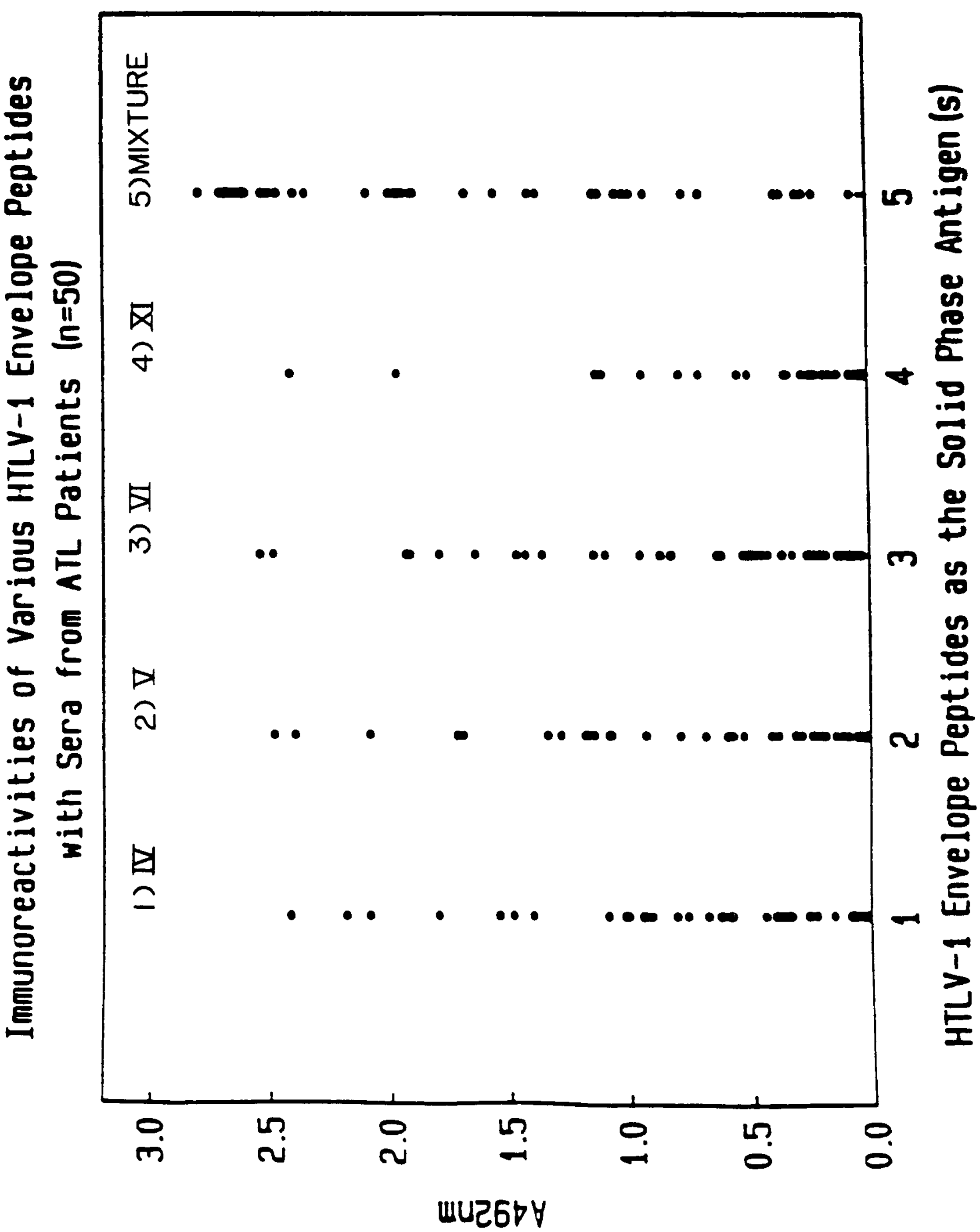
Immunoreactivities of Various HTLV-1 Envelope Peptides
with Sera from ATL Patients (n=50)
A492nm
3.0
2.5
2.0
1.5
1.0
0.5
0.0
1) IV
2) V
3) VI
4) XI
5) MIXTURE
1
2
3
4
5
HTLV-1 Envelope Peptides as the Solid Phase Antigen(s)

FIG. 3

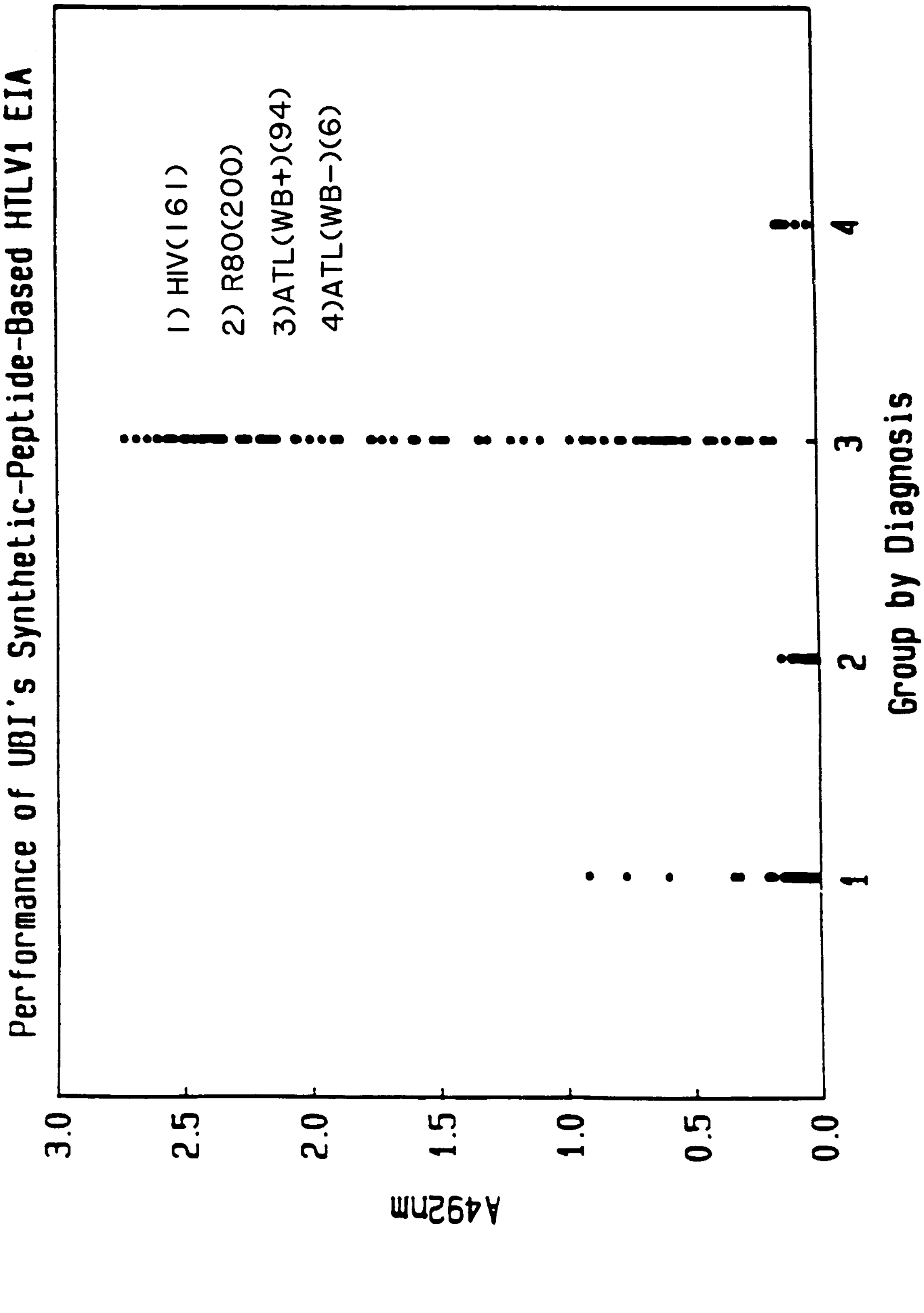
Performance of UBI's Synthetic-Peptide-Based HTLV1 EIA
1) HIV(161)
2) R80(200)
3)ATL(WB+)(94)
4)ATL(WB-)(6)
A492nm
3.0
2.5
2.0
1.5
1.0
0.5
0.0
1
2
3
4
Group by Diagnosis

FIG. 4

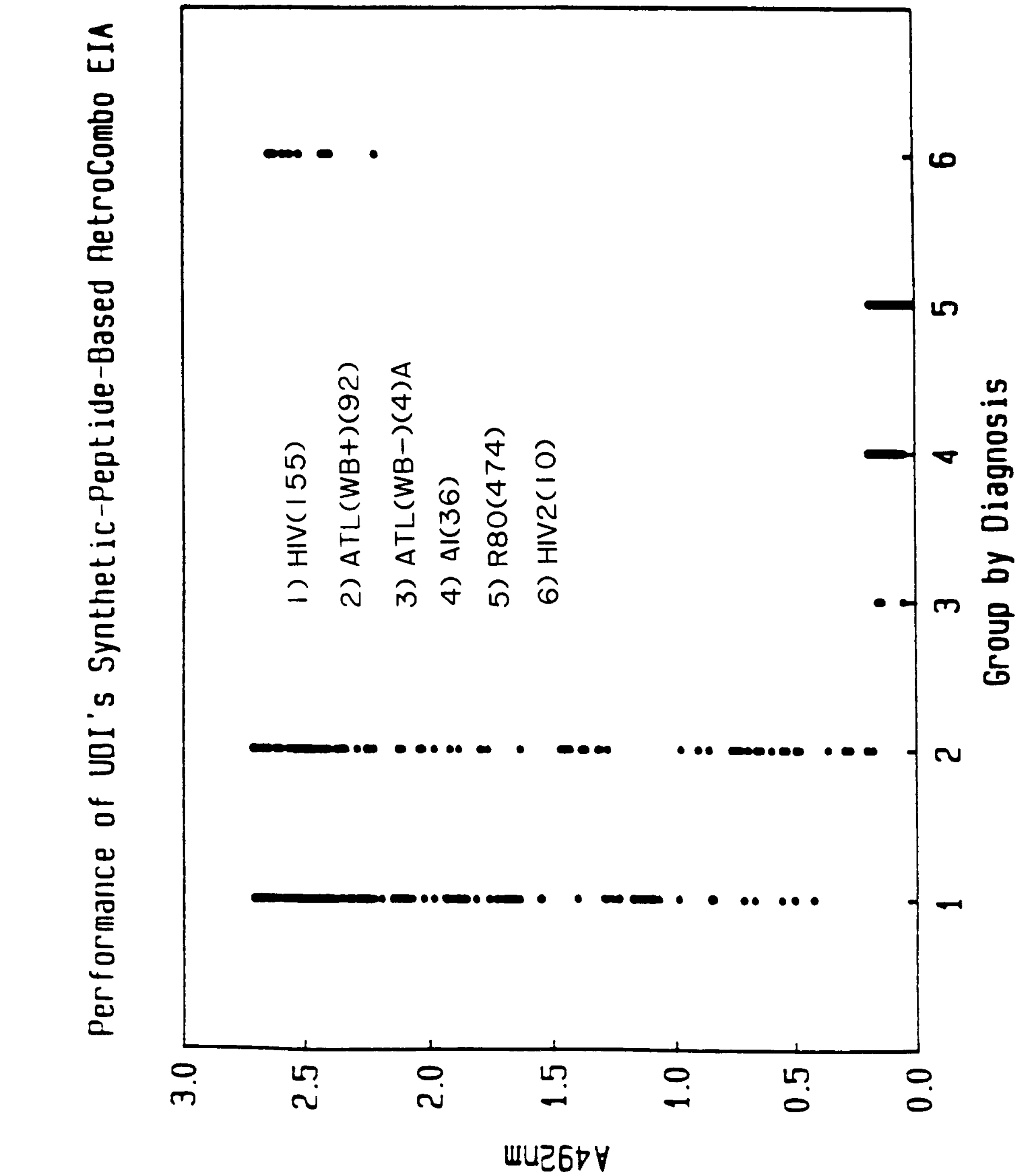
Performance of UDI's Synthetic-Peptide-Based RetroCombo EIA
A492nm
3.0
2.5
2.0
1.5
1.0
0.5
0.0
1) HIV(155)
2) ATL(WB+)(92)
3) ATL(WB-)(4)A
4) AI(36)
5) R80(474)
6) HIV2(10)
1
2
3
4
5
6
Group by Diagnosis

FIG. 5